# EUROPEAN PATENT SPECIFICATION

(11) **EP 1 532 127 B1**
(45) Date of publication and mention of the grant of the patent: **27.09.2006**
(21) Application number: 03762054.9
(22) Date of filing: 25.06.2003
(51) Int. Cl.: C07D 307/26, C07D 493/04

(54) **PREPARATION OF STEREOISOMERS OF (3ALPHA, 3ALPHA/BETA, 6ALPHA/BETA) HEXAHYDROFURO(2,3-b) FURAN-3-OL**
HERSTELLUNG VON STEREOISOMEREN VON (3ALPHA, 3ALPHA/BETA, 6ALPHA/BETA)HEXAHYDROFURO¬2,3-b|FURAN-3-OL
PREPARATION DE STEREOISOMERES DE (3ALPHA, 3ALPHA/BETA, 6ALPHA/BETA) HEXAHYDROFURO(2,3-b) FURAN-3-OL

(30) Priority: 27.06.2002 US 392677 P
(43) Date of publication of application: 25.05.2005
(73) Proprietor: SMITHKLINE BEECHAM CORPORATION, Philadelphia, PA 19101 (US)
(72) Inventor: DOAN, Brian, Daniel GlaxoSmithKline, King of Prussia, PA 19406 (US); PATTERSON, Daniel, Edward GlaxoSmithKline, Research Triangle Park, NC 27709 (US); ROBERTS, John, C. GlaxoSmithKline, Research Triangle Park, NC 27709 (US)
(74) Representative: Crawley, Karen Anne
(86) International application number: PCT/US2003/020094
(87) International publication number: WO 2004/002975

(56) References cited:
- WO-A-02/060905
- WO-A-03/022853
- WO-A-03/024974
- MAYER S ET AL: "Synthesis of Perhydro-Furo[2,3-b]Pyran (and Furan)-3-yl Methanols by Oxygenative Radical Cyclization" TETRAHEDRON, ELSEVIER SCIENCE PUBLISHERS, AMSTERDAM, NL, vol. 54, no. 30, 23 July 1998 (1998-07-23), pages 8753-8770, XP004124043 ISSN: 0040-4020
- GHOSH A K ET AL: "Nonpeptidal P2 Ligands for HIV Protease Inhibitors: Structure-Based Design, Synthesis, and Biological Evaluation" JOURNAL OF MEDICINAL CHEMISTRY, AMERICAN CHEMICAL SOCIETY. WASHINGTON, US, vol. 39, no. 17, 1996, pages 3278-3290, XP002241908 ISSN: 0022-2623 cited in the application
- UCHIYAMA M ET AL: "Stereoselective synthesis of optically active perhydrofuro[2,3-b]furan derivatives" TETRAHEDRON LETTERS, ELSEVIER SCIENCE PUBLISHERS, AMSTERDAM, NL, vol. 42, no. 28, 9 July 2001 (2001-07-09), pages 4653-4656, XP004245771 ISSN: 0040-4039
- GHOSH A K ET AL: "Synthesis and optical resolution of high affinity P2-ligands for HIV-1 protease inhibitors" TETRAHEDRON LETTERS, ELSEVIER SCIENCE PUBLISHERS, AMSTERDAM, NL, vol. 36, no. 4, 23 January 1995 (1995-01-23), pages 505-508, XP004028779 ISSN: 0040-4039

## Description

### BACKGROUND OF THE INVENTION

The human immunodeficiency virus ("HIV") is the causative agent of acquired immunodeficiency syndrome ("AIDS"), a disease characterized by the destruction of the immune system, particularly of CD4⁺ T-cells, with attendant susceptibility to opportunistic infections, and its precursor AIDS-related complex ("ARC"), a syndrome characterized by symptoms such as persistent generalized lymphadenopathy, fever and weight loss.

Among the drugs currently used to treat HIV infections in humans are those that inhibit the HIV aspartyl protease enzyme. Drugs that are used as protease inhibitors are, in general, chemically complex and are difficult to prepare in a cost-effective and efficient manner. As a result of the inherent complexity of these molecules, new and more efficient methods for their preparation are of value.

The present invention concerns processes for the preparation of four stereoisomers of (3α, 3aβ, 6aβ)hexahydrofuro[2,3-*b*]furan-3-ol, compounds useful in the preparation of inhibitors of HIV aspartyl protease, and their separation to provide single diastereoisomers in highly enantio-enriched form.

The present invention also features chemical compounds useful as intermediates in the preparation of compounds that may function as inhibitors of HIV aspartyl protease.

In WO 94/26749, Ghosh, et. al. discloses the preparation of hexahydrofuro[2,3-*b*]furan-3-ol and its use in the preparation of compounds that are effective inhibitors of HIV aspartyl protease. The method disclosed for the preparation of the hexahydrofuro[2,3-*b*]furan-3-ol sub-unit was a multi-step procedure that required the initial preparation of an enantiomerically pure starting material, followed by six additional chemical steps.

The synthesis of this sub-unit was also described by Ghosh, et. al. in Nonpeptidal P₂ Ligands for HIV Protease Inhibitors: Structure-Based Design, Synthesis and Biological Evaluation, *J*. *Med. Chem.* 1996, 39(17), p. 3278.

The present method features the use of inexpensive, nonchiral starting materials, and does not rely on the use of heavy metals or photochemistry.

### BRIEF DESCRIPTION OF THE INVENTION

The present invention provides processes and compounds that are useful in the preparation of (3α, 3aβ, 6aβ)-hexahydrofuro[2,3-*b*]furan-3-ol, diastereoisomers, and enantiomers thereof, which are useful in the preparation of compounds that may be inhibitors of HIV aspartyl protease.

### DETAILED DESCRIPTION OF THE INVENTION

The present invention provides processes and compounds that are useful in the preparation of (3α, 3aβ, 6aβ)-hexahydrofuro[2,3-*b*]furan-3-ol, diastereoisomers and enantiomers thereof. Schemes A - C and I - IX illustrate processes of the present invention.

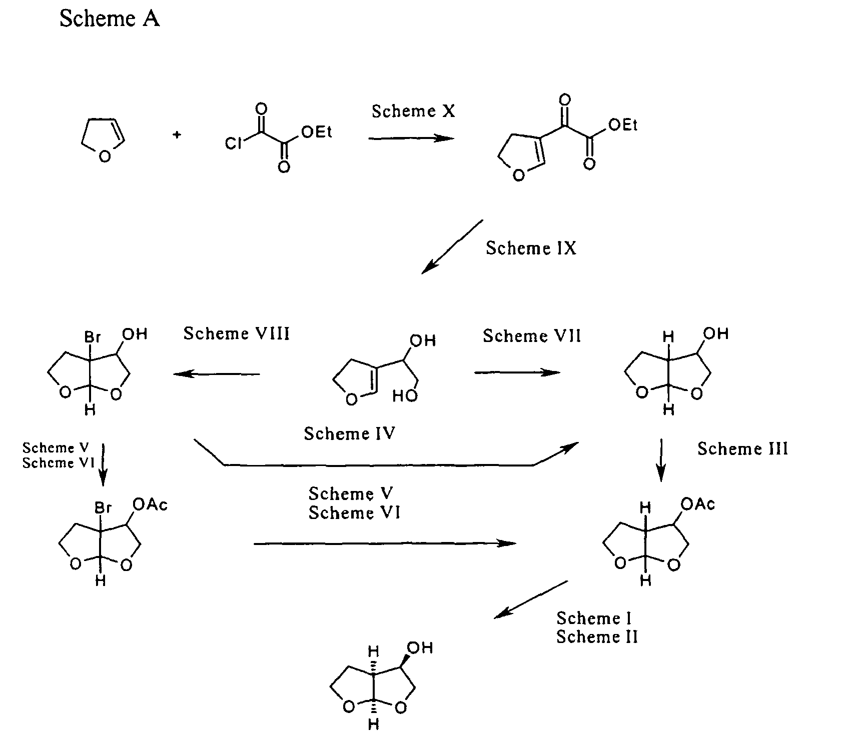

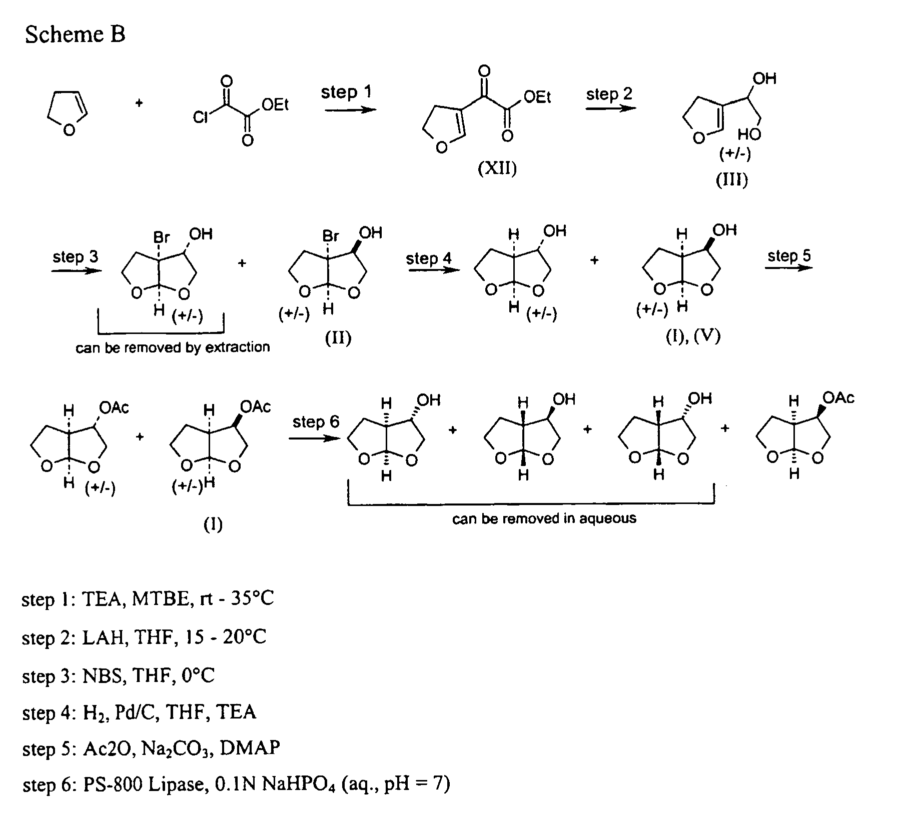

The products of step 3 may be separated before proceeding with step 4 or step 4 may be performed in the presence of the four stereoisomeric products of step 3.

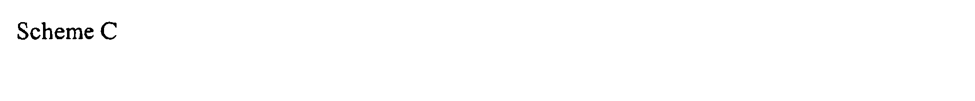

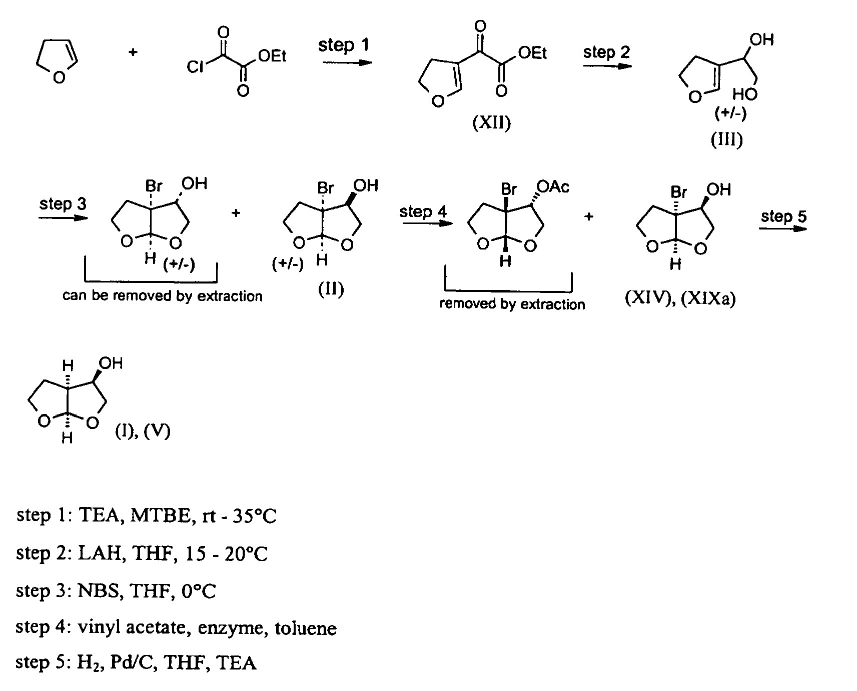

The diastereoisomers of step 3 may be separated before proceeding with step 4 or the downstream products may be separated subsequent to step 4.

The present invention features a process for the preparation of compounds of formula (I) 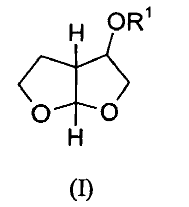 diastereoisomers, enantiomers, and mixtures thereof,
wherein R¹ is hydrogen comprising:
a) treating a compound of formula (XII) 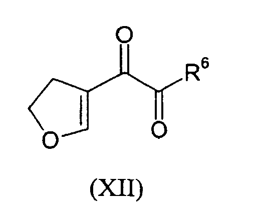 wherein:
   R⁶ is halogen, -OR⁷, or -NR⁸R⁹;
   R⁷ is hydrogen, C₁₋₆alkyl, C₃₋₈cycloalkyl, C₆₋₁₄aryl, or C₆₋₁₄arylC₁₋₆alkyl; and
   R⁸ and R⁹ are independently selected from hydrogen, C₁₋₆alkyl, C₃₋₈cycloalkyl, C₆₋₁₄aryl, and C₆₋₁₄arylC₁₋₆alkyl;
   with a first reducing agent to form an alcohol of formula (III) 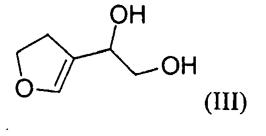
b) treating the alcohol with bromine, iodine, iodine monochloride, an N-fluoro bis sulfonamide or a compound of formula (XIII) 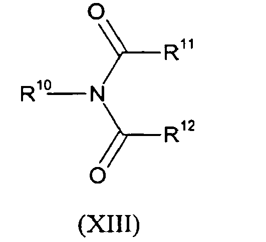 wherein:
   R¹⁰ is chlorine, bromine, or iodine; and
   R¹¹ and R¹² are independently selected from C₁₋₆alkyl, C₃₋₈cycloalkyl, C₆₋₁₄aryl, and C₆₋₁₄arylC₁₋₆alkyl, or R¹¹ and R¹² together with the atoms to which they are attached form a 5-8 membered ring;
   to form a compound of formula (II) 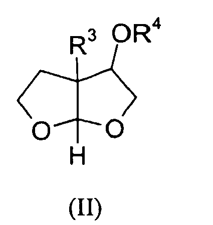 wherein R³ is halogen, and R⁴ is hydrogen;
c) treating a compound of formula (II) with a second reducing agent to afford a compound of formula (I), wherein R¹ is hydrogen.

The process described above may be used for the preparation of compounds of formula (I) wherein R¹ is hydrogen, R⁶ is -OR⁷ wherein R⁷ is C₁₋₆alkyl, and R¹⁰ - R¹² are as hereinbefore defined to yield a compound of formula (I) wherein R¹ is hydrogen. The resulting compound may optionally be separated or resolved to yield a single stereoisomer of the compound of formula (I) wherein R¹ is hydrogen.

The process described above may be used for the preparation of compounds of formula (I) wherein R¹ is -C(O)R² wherein R² is C₁₋₆alkyl, C₃₋₈cycloalkyl, C₆₋₁₄aryl, or C₆₋₁₄arylC₁₋₆alkyl; R⁶ is halogen, -OR⁷, or-NR⁸R⁹; R⁷ is hydrogen, C₁₋₆alkyl, C₃₋₈cycloalkyl, C₆₋₁₄aryl, or C₆₋₁₄arylC₁₋₆alkyl; R⁸ and R⁹ are independently selected from hydrogen, C₁₋₆alkyl, C₃₋₈cycloalkyl, C₆₋₁₄aryl, and C₆₋₁₄arylC₁₋₆alkyl; and R¹⁰ - R¹² are as hereinbefore defined to yield a compound of formula (I) wherein R¹ is hydrogen. The compounds thus formed may be converted to compounds of formula (I) wherein R¹ is -C(O)R² wherein R² is C₁₋₆alkyl, C₃₋₈cycloalkyl, C₆₋₁₄aryl, or C₆₋₁₄arylC₁₋₆alkyl by reaction, for example, with acetic anhydride in tetrahydrofuran, in the presence of sodium carbonate and DMAP. The resulting compound may optionally be separated and/or resolved to yield a single stereoisomer of the compound of formula (I) wherein R¹ is C(O)R² and R² is C₁₋₆alkyl, C₃₋₈cycloalkyl, C₆₋₁₄aryl, or C₆₋₁₄arylC₁₋₆alkyl or compounds of formula (I) wherein R¹ is hydrogen. Resolution may be accomplished by treatment with lipase or other methods known to those skilled in the art.

The process described above may be used for the preparation of compounds of formula (I) wherein R¹ is -C(O)R² wherein R² is C₁₋₆alkyl, C₃₋₈cycloalkyl, C₆₋₁₄aryl, or C₆₋₁₄arylC₁₋₆alkyl; R⁶ is -OR⁷ wherein R⁷ is C₁₋₆alkyl, C₃₋₈cycloalkyl, C₆₋₁₄aryl, or C₆₋₁₄arylC₁₋₆alkyl; and R¹⁰ - R¹² are as hereinbefore defined to yield a compound of formula (I) wherein R¹ is hydrogen. The compounds thus formed may be converted to compounds of formula (I) wherein R¹ is -C(O)R² wherein R² is C₁₋₆alkyl, C₃₋₈cycloalkyl, C₆₋₁₄aryl, or C₆₋₁₄arylC₁₋₆alkyl by reaction, for example, with acetic anhydride in tetrahydrofuran, in the presence of sodium carbonate and DMAP. The resulting compound may optionally be separated and/or resolved to yield a single stereoisomer of the compound of formula (I) wherein R¹ is C(O)R² and R² is C₁₋₆alkyl, C₃₋₈cycloalkyl, C₆₋₁₄aryl, or C₆₋₁₄arylC₁₋₆alkyl or compounds of formula (I) wherein R¹ is hydrogen. Resolution may be accomplished by treatment with lipase or other methods known to those skilled in the art.

The present invention also features processes for the preparation of compounds of formula (I) as described above, wherein the first reducing agent is selected from the group consisting of di-*iso*butylaluminum hydride (DIBAL), sodium borohydride, and lithium aluminum hydride; R⁶ in the compound of formula (XII) is -OR⁷ wherein R⁷ is C₁₋₆alkyl; the compound of formula (XIII) is N-bromosuccinimide; and the second reducing agent is palladium on carbon in combination with hydrogen. The first reducing agent is advantageously lithium aluminum hydride.

The term "C₁₋₆alkyl," alone or in combination with any other term, refers to a straight-chain or branched-chain saturated aliphatic hydrocarbon radical containing the specified number of carbon atoms. Examples of alkyl radicals include, but are not limited to, methyl, ethyl, n-propyl, isopropyl, n-butyl, isobutyl, sec-butyl, tert-butyl, pentyl, isoamyl, n-hexyl and the like.

The term "C₃₋₈cycloalkyl," alone or in combination with any other term, refers to a saturated or partially saturated carbocyclic ring composed of 3-8 carbons in any chemically stable configuration. Examples of suitable carbocyclic groups include cyclopropyl, cyclobutyl, cyclopentyl, cyclohexyl, cycloheptyl, cyclooctyl, and cyclohexenyl.

The term "C₆₋₁₄aryl" alone or in combination with any other term, refers to a carbocyclic aromatic moiety (such as phenyl or naphthyl) containing the specified number of carbon atoms, preferably from 6-14 carbon atoms, and more preferably from 6-10 carbon atoms. Examples of aryl radicals include, but are not limited to phenyl, naphthyl, indenyl, indanyl, azulenyl, fluorenyl, anthracenyl and the like.

The term "halogen" refers to fluorine, chlorine, bromine or iodine.

The term "substantially free from" in reference to a particular compound means that the compound contains from about 0 to 10% of other stereoisomeric compounds such as enantiomers and diastereoisomers.

The term "reducing agent" refers to a chemical reagent or combination of reagents known to those skilled in the art to be capable of reducing a carbonyl-containing compound. Among such reagents or combination of reagents are lithium aluminum hydride, di-*iso*butyl aluminum hydride, and sodium borohydride.

The following abbreviations may be used in the specification:
- g (grams);: mg (milligrams);
- L (liters);: mL (milliliters);
- µL (microliters);: psi (pounds per square inch);
- M (molar);: mM (millimolar);
- i. v. (intravenous);: Hz (Hertz);
- MHz (megahertz);: mol (moles);
- mmol (millimoles);: rt (room temperature);
- min (minutes);: h (hours);
- mp (melting point);: TLC (thin layer chromatography);
- Tᵣ (retention time);: RP (reverse phase);
- MeOH (methanol);: *i*-PrOH (isopropanol);
- TEA (triethylamine);: TFA (trifluoroacetic acid);
- TFAA (trifluoroacetic anhydride);: THF (tetrahydrofuran);
- DMSO (dimethylsulfoxide);: AcOEt (ethyl acetate);
- DME (1,2-dimethoxyethane);: DCM (dichloromethane);
- DCE (dichloroethane);: DMF (*N,N*-dimethylformamide);
- DMPU (*N,N'*-dimethylpropyleneurea);: CDI (1,1-carbonyldiimidazole);
- IBCF (isobutyl chloroformate);: HOAc (acetic acid);
- HOSu (*N*-hydroxysuccinimide);: HOBT (1-hydroxybenzotriazole);
- mCPBA (meta-chloroperbenzoic acid;: EDC (ethylcarbodiimide hydrochloride);
- BOC (*tert*-butyloxycarbonyl);: FMOC (9-fluorenylmethoxycarbonyl);
- DCC (dicyclohexylcarbodiimide);: CBZ (benzyloxycarbonyl);
- Ac (acetyl);: atm (atmosphere);
- TMSE (2-(trimethylsilyl)ethyl);: TMS (trimethylsilyl);
- TIPS (triisopropylsilyl);: TBS (*t*-butyldimethylsilyl);
- DMAP (4-dimethylaminopyridine);: NBS (N-bromosuccinimide);
- ATP (adenosine triphosphate);: DIBAL (di-isobutylaluminum hydride);
HPLC (high pressure liquid chromatography);
BOP (bis(2-oxo-3-oxazolidinyl)phosphinic chloride);
TBAF (tetra-*n*-butylammonium fluoride);
HBTU (O-Benzotriazole-1-yl-N,N,N',N'- tetramethyluronium hexafluorophosphate).
LAH (lithium aluminum hydride);
DPPA (diphenylphosphoryl azide);
fHN0₃ (fumed HNO₃); and
EDTA (ethylenediaminetetraacetic acid).

The compounds according to the invention may contain one or more asymmetric atoms and thus occur as racemates, racemic mixtures, single enantiomers, diastereoisomeric mixtures and individual diastereoisomers. All such isomeric forms of these compounds are expressly included in the present invention. Each stereogenic atom may be of the R or S configuration. Although the specific compounds exemplified in this application may be depicted in a particular stereochemical configuration, compounds having either the opposite stereochemistry at any given chiral center or mixtures thereof are also envisioned. When a compound of formula (I), (II), or (III) is desired as a single enantiomer, it may be obtained either by separation of diastereoisomers and/or resolution of (I), (II), (III) or by stereospecific synthesis using methods known to those skilled in the art. See, for example, Stereochemistry of Organic Compounds by E.L. Eliel and S.H. Wilen (Wiley Interscience, 1994).

Compounds containing one or more stereocenters may be drawn using plain lines to depict bonds from said stereocenters. Such structures are intended to represent both possible configurations at that center. Where the compound contains only one stereocenter and a plain line is used to depict the bond from said stereocenter, that structure is meant to depict both enantiomers. Where the compound depicted contains more than one stereocenter and is drawn using plain lines, it is meant to represent all possible stereoisomers, including all enantiomers and all diastereoisomers, and mixtures thereof. For example, compound (XIV) depicted below is meant to represent four diastereoisomers: (3*S*,3a*R*,6a*R*)-3a-bromohexahydrofuro[2,3-*b*]furan-3-ol, (3*R*,3a*R*,6a*R*)-3a-bromohexahydrofuro[2,3-*b*]furan-3-ol, (3*S*,3a*S*,6a*S*)-3a-bromohexahydrofuro[2,3-*b*]furan-3-ol, and (3*R*,3a*S*,6a*S*)-3a-bromohexahydrofuro[2,3-*b*]furan-3-ol, and mixtures thereof.

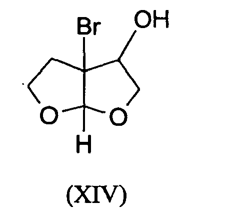

In contrast, when compounds containing stereocenters are depicted using dotted and/or bold lines, it is meant to depict the relative stereochemistry of the compound. For example, compound (XIXa) shown below is meant to represent two enantiomers: (3*S*,3a*R*,6a*R*)-3a-bromohexahydrofuro[2,3-*b*]furan-3-ol and (3*R*,3a*S*,6a*S*)-3a-bromohexahydrofuro[2,3-*b*]furan-3-ol, and mixtures thereof.

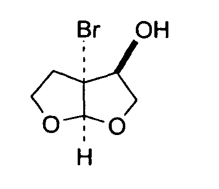

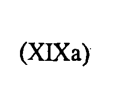

When compounds containing stereocenters are depicted using dashed or bold "wedges," they are meant to represent compounds of absolute stereochemistry. For example, compound (XX), as depicted below using dashed and bold wedges, is meant to represent a single enantiomer: (3*S*,3a*R*,6a*R*)-3a-bromohexahydrofuro[2,3-*b*]furan-3-ol, one enantiomer, either in pure form or substantially free from the opposite enantiomer, and other diastereoisomers.

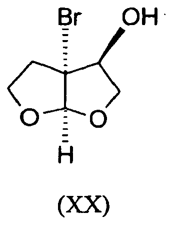

The present invention features a process for the preparation of compounds of formula (I) as described above, diastereoisomers, enantiomers, and mixtures thereof, said process comprising the step of reducing a compound of formula (XII) to afford an alcohol of formula (III).

The present invention also features a process for the preparation of compounds of formula (I), said process comprising the step of treating a compound of formula (III), 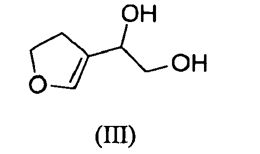 with an acid selected from hydrochloric acid, hydrobromic acid, hydroiodic acid, acetic acid, sulfuric acid, sulfonic acids such as *para*-toluenesulfonic acid, and the like.

Another feature of the present invention is a process for the preparation of a compound of formula (V) 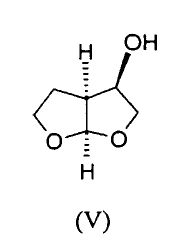 substantially free from other diastereoisomers, comprising:
a) treating a compound of formula (XII) 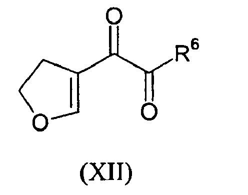 wherein:
   R⁶ is halogen, -OR⁷, or -NR⁸R⁹;
   R⁷ is hydrogen, C₁₋₆alkyl, C₃₋₈cycloalkyl, C₆₋₁₄aryl, or C₆₋₁₄arylC₁₋₆alkyl; and
   R⁸ and R⁹ are independently selected from hydrogen, C₁₋₆alkyl, C₃₋₈cycloalkyl, C₆₋₁₄aryl, and C₆₋₁₄arylC₁₋₆alkyl;
   with a first reducing agent to form an alcohol of formula (III) 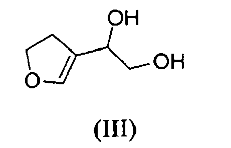
b) treating the alcohol with bromine, iodine, iodine monochloride, an N-fluoro bis sulfonamide or a compound of formula (XIII) 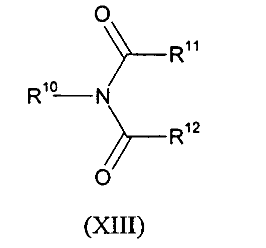 wherein:
   R¹⁰ is chlorine, bromine, or iodine; and
   R¹¹ and R¹² are independently selected from C₁₋₆alkyl, C₃₋₈cycloalkyl, C₆₋₁₄aryl, and C₆₋₁₄arylC₁₋₆alkyl, or R¹¹ and R¹² together with the atoms to which they are attached form a 5-8 membered ring;
   to form a compound of formula (II) 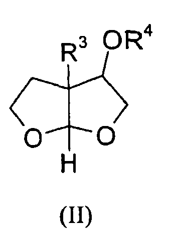 wherein R³ is halogen and R⁴ is hydrogen;
c) treating a compound of formula (II) with a second reducing agent to afford a compound of formula (V).

Also featured in the present invention are intermediate compounds of formula (II) 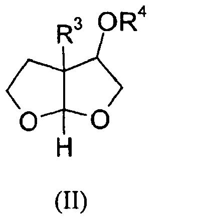 wherein:
R³ is halogen;
R⁴ is hydrogen or -C(O)R⁵;
R⁵ is hydrogen, C₁₋₆alkyl, C₃₋₈cycloalkyl, C₆₋₁₄aryl, or C₆₋₁₄arylC₁₋₆alkyl; and
diastereisomers, enantiomers, and mixtures thereof.

Particularly advantageous are compounds of formula (II), wherein R³ is bromine and R⁴ is hydrogen, and compounds of formula (II) wherein R³ is bromine and R⁴ is -C(O)R⁵ and R⁵ is C₁₋₆alkyl, preferably -CH₃.

Compounds of formula (II) wherein R³ is halogen; R⁴ is hydrogen or -C(O)R⁵; R⁵ is hydrogen, C₁₋₆alkyl, C₃₋₈cycloalkyl, C₆₋₁₄aryl, or C₆₋₁₄arylC₁₋₆alkyl; diastereoisomers, enantiomers, and mixtures thereof, made be made by treating a compound of formula (XII) wherein R⁶ - R⁹ are as hereinbefore defined, with a reducing agent to form an alcohol of formula (III), treating the alcohol of formula (III) with bromine, iodine, iodine monochloride, or the like, or a compound of formula (XIII) wherein R¹⁰ - R¹² are as hereinbefore defined, to form a compound of formula (II) as defined above. Compounds of formula (II) may be optionally resolved by treatment with lipase such that the acetate is the desired enantiomer followed by either reduction/hydrolysis or hydrolysis/reduction, as shown below. 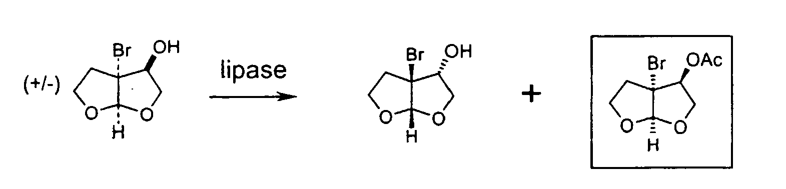 Alternatively, if the alcohol is the desired enantiomer, a compound of formula (II) may be resolved by treatment with lipase followed by reduction.

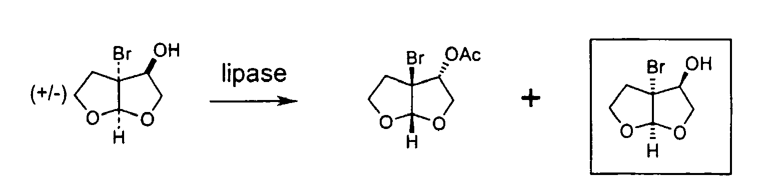

The present invention also features an intermediate compound of formula (XIX) 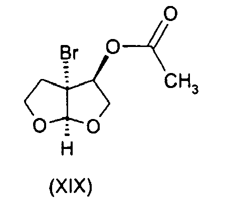 as a mixture with its stereoisomeric compounds, or substantially free from other stereoisomeric compounds.

The compound of formula (XIX), or its stereoisomers or any mixture thereof, may be made by treating a compound of formula (XII) wherein R⁶ is halogen, -OR⁷, or -NR⁸R⁹; R⁷ is hydrogen, C₁₋₆alkyl, C₃₋₈cycloalkyl, C₆₋₁₄aryl, or C₆₋₁₄arylC₁₋₆alkyl; and R⁸ and R⁹ are independently selected from hydrogen, C₁₋₆alkyl, C₃₋₈cycloalkyl, C₆₋₁₄aryl, and C₆₋₁₄arylC₁₋₆alkyl; with a reducing agent to form an alcohol of formula (III), treating the alcohol with a compound of formula (XIII) wherein R¹⁰ is chlorine, bromine, or iodine; and R¹¹ and R¹² are independently selected from C₁₋₆alkyl, C₃₋₈cycloalkyl, C₆₋₁₄aryl, and C₆₋₁₄arylC₁₋₆alkyl, or R¹¹ and R¹² together with the atoms to which they are attached form a 5-8 membered ring to yield a compound of formula (XIX).

Included in the present invention is the intermediate compound of formula (XX) 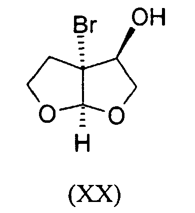 as a mixture with its stereoisomeric compounds, or substantially free from other stereoisomeric compounds.

Compounds of formula (XX) may be prepared by treating a compound of formula (XII) wherein R⁶ is halogen, -OR⁷, or -NR⁸R⁹; R⁷ is hydrogen, C₁₋₆alkyl, C₃₋₈cycloalkyl, C₆₋₁₄aryl, or C₆₋₁₄arylC₁₋₆alkyl; and R⁸ and R⁹ are independently selected from hydrogen, C₁₋₆alkyl, C₃₋₈cycloalkyl, C₆₋₁₄aryl, and C₆₋₁₄arylC₁₋₆alkyl; with a reducing agent to form an alcohol of formula (III), treating the alcohol with N-bromosuccinimide to form a compound of formula (XX) and optionally resolving to yield diastereoisomers of compounds of formula (XX).

The present invention also features 1-(4,5-dihydrofuran-3-yl)ethane-1,2-diol, a compound of formula (III)

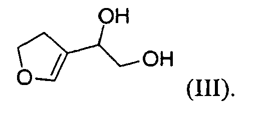

Compound (III) may be prepared by treating a compound of formula (XII) wherein R⁶ is halogen, -OR⁷, or -NR⁸R⁹; where R⁷ is hydrogen, C₁₋₆alkyl, C₃₋₈cycloalkyl, C₆₋₁₄aryl, or C₆₋₁₄arylC₁₋₆alkyl; and R⁸ and R⁹ are independently selected from hydrogen, C₁₋₆alkyl, C₃₋₈cycloalkyl, C₆₋₁₄aryl, and C₆₋₁₄arylC₁₋₆alkyl; with a reducing agent.

In the process for the preparation of a compound of formula (III) as described above, the reducing agent may be selected from the group consisting of di-*iso*butylaluminum hydride (DIBAL), sodium borohydride, and lithium aluminum hydride. The reducing agent is advantageously lithium aluminum hydride.

In the processes of the present invention described above, the first reducing agent may be selected from hydride-delivery reagents that include di-*iso*butylaluminum hydride (DIBAL), sodium borohydride, lithium aluminum hydride and the like.

In the processes of the present invention described above for the preparation of compounds of formula I, II, V, XIX, XIV and XX, the first reducing agent may be selected from hydride-delivery reagents that include di-*iso*butylaluminum hydride (DIBAL), sodium borohydride, lithium aluminum hydride and the like; R⁶ in the compound of formula (XII) may be -OR⁷ where R⁷ may be C₁₋₆alkyl; the compound of formula (XIII) may be N-bromosuccinimide; and the second reducing agent may be palladium on carbon in combination with hydrogen. The first reducing agent is advantageously lithium aluminum hydride.

In the processes of the present invention the second reducing agent may be selected from reagents or combination of reagents known to those skilled in the art to reduce carbon-halogen bonds. Among these reagents or combination of reagents are 10% w/w palladium on carbon in combination with ammonium formate, 5% w/w palladium on carbon in combination with ammonium formate, 5% w/w palladium on carbon in combination with formic acid, 10% w/w palladium on carbon in combination with formic acid, 10% w/w palladium on carbon in combination with hydrogen, Raney Nickel in combination with hydrogen, or preferably 5% w/w palladium on carbon in combination with hydrogen.

Compounds of formula (I), wherein R¹ is hydrogen, and diastereoisomers, enantiomers, and mixtures thereof, can be prepared from compounds of formula (I), wherein R¹ is - C(O)R², and R² is as hereinbefore defined, by reaction with a reagent or combination of reagents known to those skilled in the art to be basic. Among such reagents or combination of reagents are lithium hydroxide, sodium hydroxide, potassium hydroxide, sodium carbonate, sodium bicarbonate, and ammonium hydroxide, preferably potassium carbonate. These reactions are typically performed in a protic solvent such as water, *tert*-butanol, ethanol, or preferably methanol, or in a mixture of solvents, preferably a mixture of dichloromethane and methanol. These reactions are performed at a temperature from 0-50 °C, preferably ambient temperature. For example, as shown in Scheme I (3*R*,3a*S*,6a*R*)-hexahydrofuro[2,3-*b*]furan-3-yl acetate (IV) was allowed to react with potassium carbonate in a mixture of methanol and dichloromethane at room temperature to afford (3R,3aS,6aR)-hexahydrofuro[2,3-*b*]furan-3-ol (V).

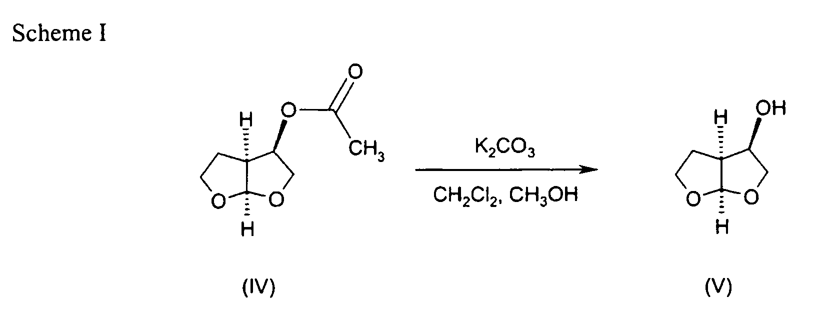

Alternatively, compounds of formula (I), wherein R¹ is hydrogen, and diastereoisomers, enantiomers, and mixtures thereof, can be prepared from compounds of formula (I), wherein R¹ is -C(O)R², and R² is as hereinbefore defined, by reaction with an appropriate esterase enzyme. A racemic mixture of esters may be allowed to react with an appropriate esterase enzyme under conditions that allow for reaction of predominantly one enantiomer of the ester to provide a mixture of diastereoisomeric alcohols and the corresponding ester of one enantiomer, substantially free of the other enantiomer and other diastereoisomers. See, E.L. Eliel and S.H. Wilen, Stereochemistry of Carbon Compounds, Wiley-Interscience, 1994, pp. 409-415. These reactions are typically performed in water as solvent and at a pH from pH = 6.8-7.2, preferably 7.0, and at a temperature from 32-50 °C, preferably 35 °C. For example, as shown in Scheme II, racemates (VI) and (VII) may be allowed to react with esterase PS-800 at a pH of 6.8-7.2 and at 32-38 °C, to afford (3*S*,3a*S*,6a*R*)-hexahydrofuro[2,3-*b*]furan-3-ol (VIII), (3*R*,3a*R*,6a*S*)-hexahydrofuro[2,3-*b*]furan-3-ol (IX), (3*S*,3a*R*,6a*S*)-hexahydrofuro[2,3-*b*]furan-3-ol (X), and (3*R*,3a*S*,6a*R*)-hexahydrofuro[2,3-*b*]furan-3-yl acetate (XI).

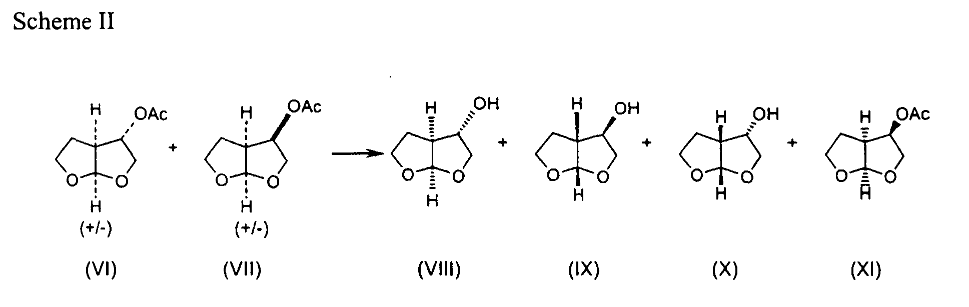

The hydrolyzed isomers (VIII, IX and X) may be washed into a water layer and the remaining ester, compound (XI) for example, may then be deprotected using mildly basic conditions as described above, for example using potassium carbonate in a mixture of dichloromethane and methanol, to afford one enantiomer of the corresponding alcohol, substantially free of the other enantiomer and other diastereoisomers.

Other methods may be used to afford one enantiomer or diastereoisomer of a compound of formula (I), substantially free from the opposite enantiomer and other diastereoisomers. First, a racemic mixture of hexahydrofuro[2,3-*b*]furan-3-ol may be resolved by converting the mixture of enantiomers into a mixture of diastereoisomers, followed by traditional methods of separation, such as silica chromatography, crystallization, or extraction. In this type of resolution, the racemic alcohol may be allowed to react with a chiral nonracemic compound (the resolving agent) resulting in the formation of a diastereoisomeric mixture. Typically, the chiral nonracemic compound is either an acid chloride, a carbamyl chloride or a chloroformate, resulting in the formation of a diastereoisomeric mixture of esters, carbamates, or carbonates, respectively. The choice of the chiral nonracemic resolving agent will depend on factors known to those skilled in the art. For example, see Eliel, et. al., p. 322.

The racemic alcohol may be allowed to react with a lipase enzyme capable of converting one enantiomer of the alcohol into an ester. The ester and the remaining alcohol may then be separated by methods known to those skilled in the art. See Eliel, et. al., p. 413.

Alternatively, the *exo* and *endo* diastereoisomers of compounds of formula (II), 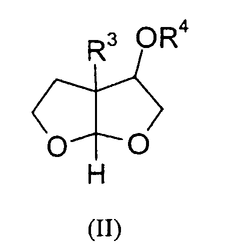 wherein R³ is halogen and R⁴ is hydrogen, may be separated from one another using chromatography, crystallization, or extraction. Methods of resolving compounds of formula (I) can be applied to the resolution of compounds of formula (II).

Compounds of formula (I), wherein R¹ is -C(O)R², and R² is as hereinbefore defined, may be prepared from compounds of formula (I), wherein R¹ is hydrogen by reaction with an appropriate esterifying agent, an acid chloride or anhydride for example. These reactions are typically performed in an aprotic solvent, tetrahydrofuran for example, and in the presence of a compound capable of acting as a base, sodium carbonate for example. In addition, a compound capable of acting as a catalyst may be advantageously used, for example 4-N,N-dimethylaminopyridine (DMAP). For example, as shown in Scheme (III), hexahydrofuro[2,3-*b*]furan-3-ol was allowed to react with acetic anhydride in tetrahydrofuran, in the presence of sodium carbonate and DMAP, to afford hexahydrofuro[2,3-*b*]furan-3-yl acetate.

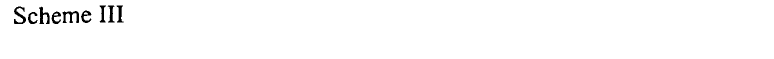

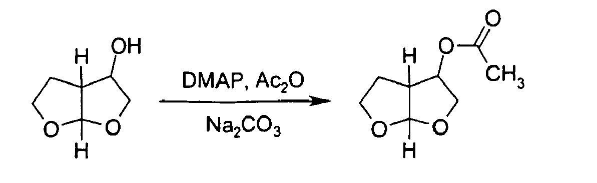

Compounds of formula (I), wherein R¹ is hydrogen, may prepared from compounds of formula (II), wherein R³ is halogen and R⁴ is hydrogen, by reaction with a reagent or combination of reagents known to those skilled in the art to reduce carbon-halogen bonds. Among these reagents or combination of reagents are 10% w/w palladium on carbon in combination with ammonium formate, 5% w/w palladium on carbon in combination with ammonium formate, 5% w/w palladium on carbon in combination with formic acid, 10% w/w palladium on carbon in combination with formic acid, 10% w/w palladium on carbon in combination with hydrogen, Raney Nickel in combination with hydrogen, or preferably 5% w/w palladium on carbon in combination with hydrogen. These reactions are typically performed in a solvent such as methanol, or preferably tetrahydrofuran (THF), in the presence of base, such as triethylamine, and at a temperature from 0-75 °C, preferably ambient temperature. For example, as shown in Scheme IV, 3a-bromohexahydrofuro[2,3-*b*]furan-3-ol (XIV) was allowed to react with hydrogen and 5% w/w palladium on carbon, in THF and in the presence of triethylamine, to afford hexahydrofuro[2,3-*b*]furan-3-ol, compound (I) wherein R¹ is hydrogen.

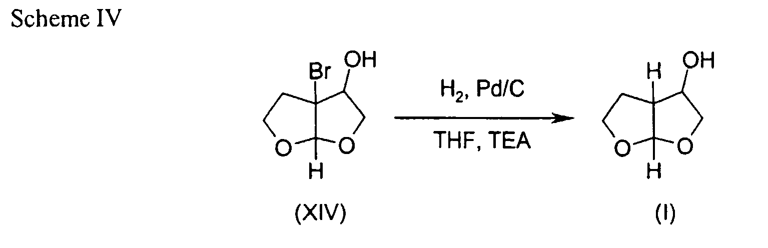

Alternatively, compounds of formula (I) wherein R¹ is hydrogen may prepared from compounds of formula (II), wherein R³ is halogen and R⁴ is hydrogen, first by conversion to a compound of formula (II), wherein R³ is halogen, R⁴ is -C(O)R⁵, and R⁵ is hydrogen, C₁₋₆alkyl, C₃₋₈cycloalkyl, C₆₋₁₄aryl, or C₆₋₁₄arylC₁₋₆alkyl, followed by reduction, and, optionally, resolution. For example, as shown in Scheme V, compound (XIV) may be allowed to react with an acylating agent followed by reduction of the bromine-carbon bond, and optionally resolution, and optionally followed by deprotection to afford predominantly (3*R*,3a*S*,6a*R*)-hexahydrofuro[2,3-*b*]furan-3-ol (XV).

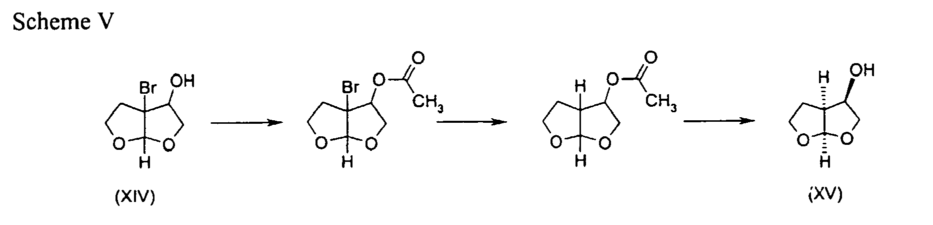

Alternatively, compounds of formula (I) wherein R¹ is hydrogen may prepared from compounds of formula (II), wherein R³ is halogen and R⁴ is hydrogen, first by conversion to a compound of formula (II), wherein R³ is halogen, R⁴ is -C(O)R⁵, and R⁵ is hydrogen, C₁₋₆alkyl, C₃₋₈cycloalkyl, C₆₋₁₄aryl, or C₆₋₁₄arylC₁₋₆alkyl, followed by resolution and reduction. As shown in Scheme VI, compound (XIV) may be allowed to react with an acylating agent affording compound (XVI), which may then be resolved to afford compound (XIX), reduced and deprotected to afford (3*R*,3a*S*,6a*R*)-hexahydrofuro[2,3-*b*]furan-3-ol (XV), substantially free of the opposite enantiomer and other diastereoisomers.

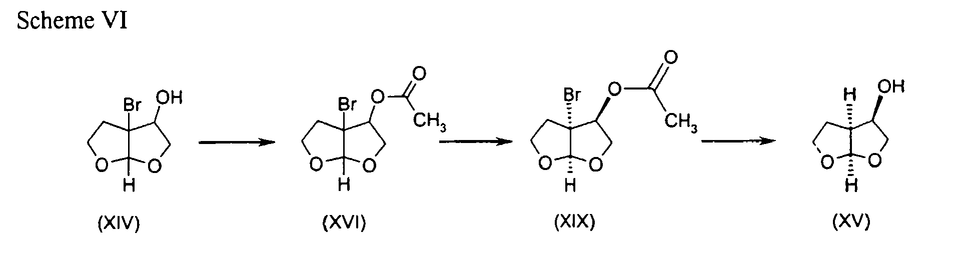

Alternatively, compounds of formula (I), wherein R¹ is hydrogen, may be prepared from compounds of formula (III) by reaction with a reagent or combination of reagents capable of acting as a proton source. Among the reagents or combination of reagents capable of acting as a proton source are Bronsted acids known to those skilled in the art, for example hydrochloric acid, hydrobromic acid, hydroiodic acid, acetic acid, sulfuric acid, and sulfonic acids such as para-toluenesulfonic acid. These reactions are typically performed in an aprotic solvent, such as tetrahydrofuran, and at a temperature from -30 to 50 °C, preferably - 10 to 10 °C. For example, as shown in Scheme VII, 1-(4,5-dihydrofuran-3-yl)ethane-1,2-diol may be allowed to react with an acid to afford hexahydrofuro[2,3-*b*]furan-3-ol.

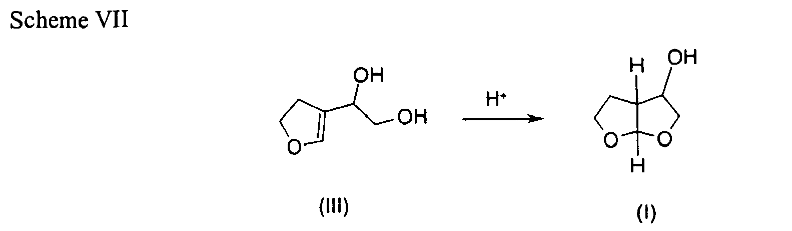

Compounds of formula (II) wherein R³ is halogen and R⁴ is hydrogen may be prepared from compounds of formula (III) by reaction with a reagent or combination of reagents capable of providing a source of electrophilic halogen. Among the reagents or combination of reagents known to provide an electrophilic source of halogen are bromine, iodine, N-iodosuccinimide, N-chlorosuccinimide, or preferably N-bromosuccinimide (NBS). These reactions are typically performed in an aprotic solvent, such as THF, and at a temperature from -78 to 50 °C, preferably from -5 to 0 °C. For example, as shown in Scheme VIII, 1-(4,5-dihydrofuran-3-yl)ethane-1,2-diol may be allowed to react with NBS in THF at-5 to 0 °C to afford 3a-bromohexahydrofuro[2,3-*b*]furan-3-ol (XIV).

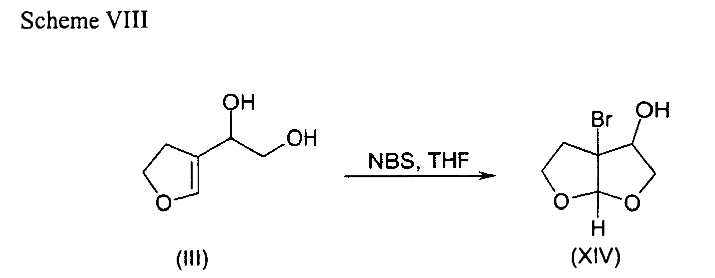

Compounds of formula (III), and enantiomers and mixtures thereof, may be prepared by reduction of compounds of formula (XII), wherein R⁶ is as hereinbefore defined. Reduction may be accomplished by allowing a compound of formula (XII) to react with a reagent or combination of reagents known to those skilled in the art as capable of reducing a compound of formula (XII). Among the reagents or combination of reagents known to those skilled in the art to be capable of such a reduction are lithium aluminum hydride (LAH), di-*iso*butylaluminum hydride (DIBAL), and sodium borohydride. These reactions are typically performed in an aprotic solvent, such as THF, and at a temperature from -78 to 60 °C, preferably 0 to 60 °C. For example, as shown in Scheme IX, compound (XVII), ethyl 4,5-dihydrofuran-3-yl(oxo)acetate, may be allowed to react with LAH in THF at 15 to 20 °C to afford compound (III).

Chiral reduction of a compound of formula (XII) may afford an enantiomerically enriched compound of formula (III), as shown below.

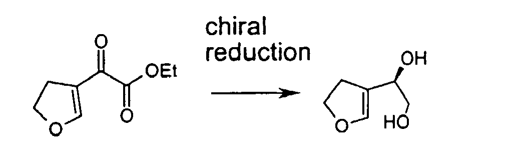

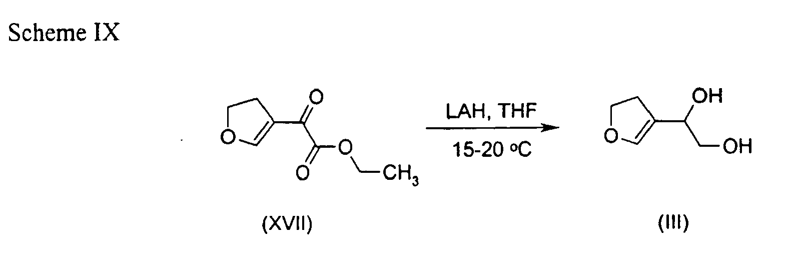

Compounds of formula (XII), wherein R⁶ is as hereinbefore defined, may be prepared by reaction of 2,3-dihydrofuran with a compound of formula (XVIII), 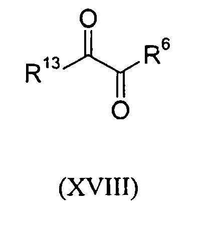 wherein:
R⁶ is as hereinbefore defined; and
R¹³ is chlorine or bromine.

These reactions may be performed in an aprotic solvent, THF for example or preferably methyl *tert*-butyl ether (MTBE), in the presence of a compound capable of acting as a base, for example pyridine, DMAP, or preferably triethylamine, and at a temperature from 0 to 80 °C, preferably ambient temperature to 35 °C. For example, as shown in Scheme X, 2,3-dihydrofuran may be allowed to react with ethyl chlorooxoacetate in MTBE, in the presence of triethylamine and at ambient temperature to 35 °C to afford compound (XVII), ethyl 4,5-dihydrofuran-3-yl(oxo)acetate.

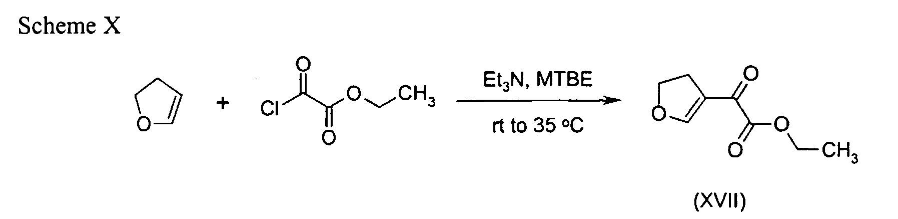

Compounds of formula (XVIII), wherein R⁶ and R¹³ are as hereinbefore defined, are commercially available or may be prepared by methods known to those skilled in the art.

Compounds of formula (I) wherein R¹ is hydrogen or -C(O)R² wherein R² is C₁₋₆alkyl, C₃₋₈cycloalkyl, C₆₋₁₄aryl, or C₆₋₁₄arylC₁₋₆alkyl may be enantiomerically enriched as illustrated by the following procedures, as more fully described hereinabove and in the examples.

The enantiomer of the acetate may be obtained by resolution of the bromo alcohol followed either reduction/hydrolysis or hydrolysis/reduction.

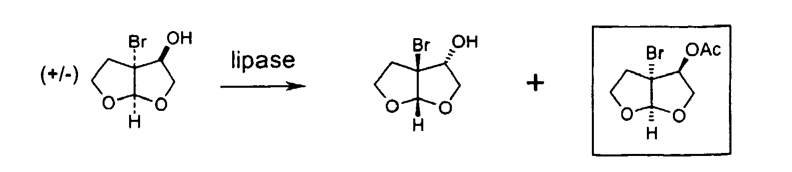

The enantiomer of the alcohol may be obtained by resolution of the bromo alcohol followed by reduction.

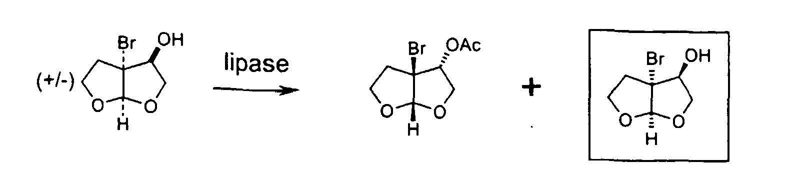

The enantiomer of the acetate may be obtained by resolution of the bromoacetate by either reduction/hydrolysis or hydrolysis/reduction.

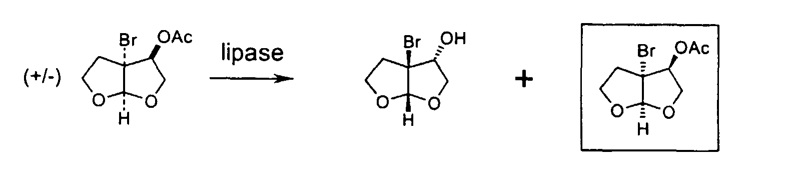

The enantiomer of the alcohol may be obtained by resolution of the acetate followed by reduction.

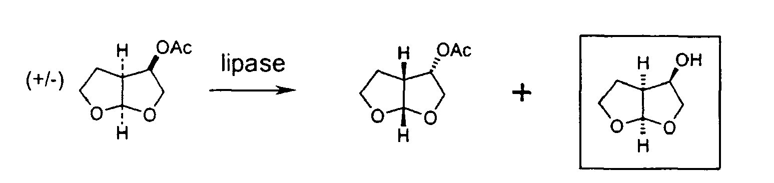

The enantiomer of the acetate may be obtained by resolution of the alcohol followed by hydrolysis.

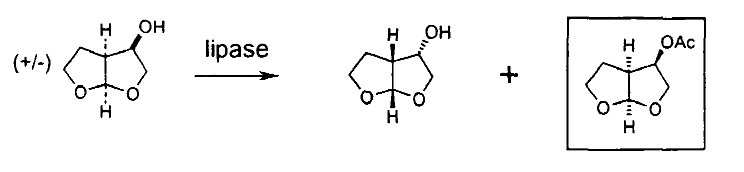

The enantiomer of the alcohol may be obtained by resolution of the alcohol.

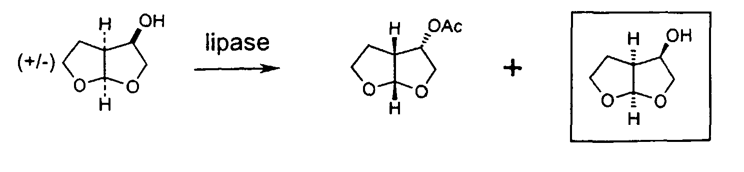

The enantiomer of the acetate may be obtained by resolution of the acetate followed by hydrolysis.

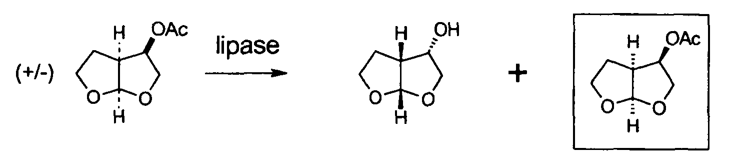

The enantiomer of the alcohol may be obtained by resolution of the acetate.

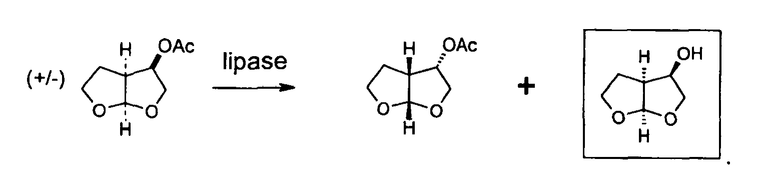

### EXAMPLES

Unless otherwise noted, all starting materials were obtained from commercial suppliers and used without further purification.

All references to ether are to diethyl ether; brine refers to a saturated aqueous solution of NaCl. Unless otherwise indicated, all temperatures are expressed in °C (degrees Centigrade). All reactions are conducted under an inert atmosphere at room temperature unless otherwise noted.

¹H NMR spectra were recorded on a Varian VXR-300, a Varian Unity-300, a Varian Unity-400 instrument, a Bruker ARX 300, or a General Electric QE-300. Chemical shifts are expressed in parts per million (ppm, δ units). Coupling constants are in units of hertz (Hz). Splitting patterns describe apparent multiplicities and are designated as s (singlet), d (doublet), t (triplet), q (quartet), m (multiplet), br (broad).

Low-resolution mass spectra (MS) were recorded on a JOEL JMS-AX505HA, JOEL SX-102, or a SCIEX-APIiii spectrometer; high resolution MS were obtained using a JOEL SX-102A spectrometer. All mass spectra were taken under electrospray ionization (ESI), chemical ionization (CI), electron impact (EI) or by fast atom bombardment (FAB) methods. Infrared (IR) spectra were obtained on a Nicolet 510 FT-IR spectrometer using a 1-mm NaCl cell. All reactions were monitored by thin-layer chromatography on 0.25 mm E. Merck silica gel plates (60F-254), visualized with UV light, 5% ethanolic phosphomolybdic acid, p-anisaldehyde solution, or ceric ammonium nitrate solution. Flash column chromatography was performed on silica gel (230-400 mesh, Merck).

### Example 1: Preparation of ethyl 4,5-dihydrofuran-3-yl(oxo)acetate

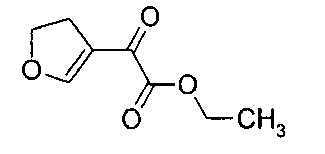

A flask was charged with 2,3-dihydrofuran (0.77wt, 1.5 eq.), triethylamine (0.82 wt, 1.1 eq.) and MTBE (4 vol). To this solution at rt was added dropwise ethyl chlorooxoacetate (1 wt., 1eq.). During the addition the temperature rose and was kept below 35 °C by external cooling (total addition time 1 h). After the addition, the reaction was allowed to cool to rt, and stirred for 2 h. The reaction mixture was washed with water (3 x 2 volumes). The organic layer was concentrated at 30 °C to afford ethyl 4,5-dihydrofuran-3-yl(oxo)acetate as an oil (76-89%).

### Example 2: Preparation of (+/-)-1-(4,5-dihydrofuran-3-yl)ethane-1,2-diol

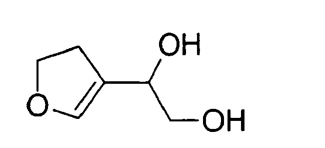

A flask fitted with an addition funnel, stirrer and nitrogen inlet was charged with lithium aluminium hydride (1M in THF, 5.8 vol, 1 eq.). The reaction was cooled to 15 °C. A solution of ethyl 4,5-dihydrofuran-3-yl(oxo)acetate (1 wt., 1 eq.) in THF (4 vol) was added dropwise keeping the temperature between 15-20 °C (1 h total addition time). After the addition was complete, the reaction was stirred at rt for 30 min and then cooled to -5°C. A 2:1 solution of THF:water (12 mL/g versus LAH, 2.66 vol) was added slowly keeping the temperature below 5 °C. Sodium hydroxide (15% aq., 4 mL/g versus LAH, 0.89 vol) was added dropwise, followed by water (12 mL/g versus LAH, 2.66 vol.). Celite (0.33 wt) was added and the resulting slurry was stirred 1 h at rt, and was filtered.

### Example 3: Preparation of 3a-bromohexahydrofuro[2,3-b]furan-3-ol

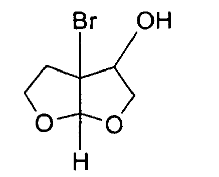

To the filtrate from Example 2 at -5 °C was added solid N-bromosuccinimide portionwise, keeping the temperature below 0 °C until the reaction was complete by GC (~30 min). The solvent (approx 10 vol) was removed in vacuo. The reaction was diluted with ethyl acetate (7 vol.) and was washed with 10% sodium sulfite (2 vol), and brine (2 x 2 vol). Triethylamine (0.005 wt) was added and the organic layer was concentrated to give approx. 3:1 mixture of diastereoisomers as an oil (45-50%). Triethylamine (0.005 wt) was added to the product as a stabilizer.

### Example 4: Preparation of hexahydrofuro[2,3-b]furan-3-ol

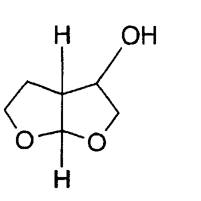

3a-bromohexahydrofuro[2,3-*b*]furan-3-ol (1 wt., 1 eq), THF (6 vol), and triethylamine (0.85 vol, 1.25 eq.) were added to a Pyrex screw-top vessel with magnetic stirbar. Degussa type E101 1 NE/W palladium on carbon (0.1 wt, 5% Pd/C, 50% water) was added to the vessel. This material is subjected to hydrogenation at 0.2 barge H₂ for 20 hours. Reaction was purged with nitrogen, and Celite (0.06 wt) was added and stirred 30 minutes. The mixture was filtered through Celite, and rinsed with THF (1.5 volumes) to afford the title compound.

### Example 5: Preparation of hexahydrofuro[2,3-b]furan-3-yl acetate

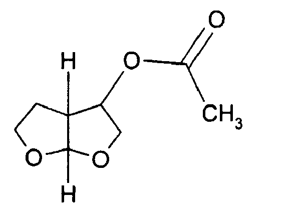

To the filtrate from Example 4 was added sodium carbonate (1.0 wt., 2 eq), and DMAP (0.01 wt., 0.02 eq). The resulting slurry was cooled to 0 °C, and acetic anhydride (0.53 wt, 1.1 eq) was added dropwise over 30 min. The reaction was warmed to rt and stirred overnight. The reaction was filtered, and the filtrate was concentrated in vacuo to an oil. The oil was dissolved in methylene chloride (5 vol) and was washed with 1N HCl (2 vol), and water (2 vol.). The organic layer was concentrated to give the title compound as an oil (90-95%).

### Example 6: Preparation of (3R,3aS,6aR)-hexahydrofuro[2,3-b]furan-3-yl acetate

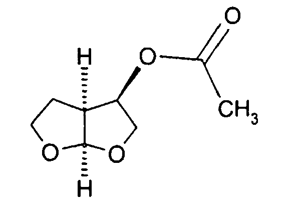

A reactor was charged with 0.1N Na₂HPO₄ (adjusted to pH=7.0 with phosphoric acid). The product from Example 5 (1eq, 1 wt) (with a pH adjustment using 15% NaOH if necessary) was added and the solution heated to 35 +/- 3 °C. PS-800 (0.0069 wt., 1000 u/mmol) was added and the pH kept between 6.8 and 7.2 with slow addition of 15% NaOH. The reaction was followed by achiral and chiral GC until all of the undesired acetates had been hydrolyzed (ca. 3-7 d). Celite (0.5 wt) was added, followed by methylene chloride (4.0 vol) and the slurry stirred for 15 min. Filtration through Celite with a methylene chloride (2.0 vol) wash gave a biphasic mixture which is separated. The organic was washed with water (3 x 1 vol) to remove the undesired alcohol diastereoisomers.

### Example 7: Preparation of (3R,3aS,6aR)-hexahydrofuro[2,3-b]furan-3-ol

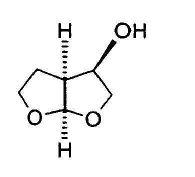

To the organic layer from Example 6 were added methanol (3 vol.), followed by potassium carbonate (0.0051 wt, 0.0064 eq.). The reaction was stirred at ambient temperature overnight. The solution was filtered and concentrated to give the title product as an oil (30-35% from 3a-bromohexahydrofuro[2,3-b]furan-3-ol when the starting diastereomeric ratio was 3: 1).

### Example 8: Preparation of hexahydrofuro[2,3-b]furan-3-ol from (+/-)-1-(4,5-dihydrofuran-3-yl)ethane-1,2-diol

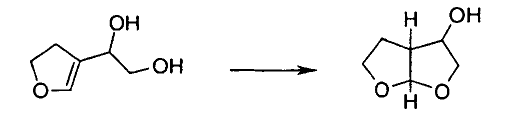

A solution of 1-(4,5-dihydrofuran-3-yl)ethane-1,2-diol (1 wt) is stirred in methanol (10 vol) and treated with catalytic p-toluenesulfonic acid. After 20 min hexahydrofuro[2,3-*b*]furan-3-ol is observed by TLC. This title product is isolated by methods known to those skilled in the art.

### Example 9: Preparation of hexahydrofuro[2,3-b]furan-3-yl acetate from 3a-bromohexahydrofuro[2,3-b]furan-3-ol

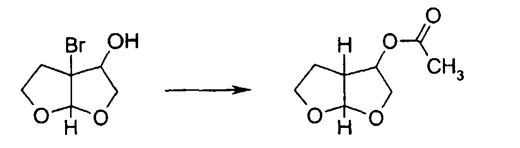

A 3:1 mixture of 3a-bromohexahydrofuro[2,3-*b*]furan-3-ol (1 wt., 1 eq), THF (6 vol), and triethylamine (1.53 vol, 2.25 eq.) is treated with with acetic anhydride (0.53 wt, 1.1 eq) and DMAP (0.01 wt, 0.02eq). The reaction is stirred for 2 h and the resulting slurry is added to a Pyrex screw-top vessel with magnetic stirbar already charged with Degussa type E101 NE/W palladium on carbon (0.1 wt, 5% Pd/C, 50% water). This mixture is subjected to hydrogenation at 0.2 barge H₂ for 20 h. The reaction mixture is purged with nitrogen, and Celite (0.06 wt) is added and stirred 30 min. The mixture is filtered through Celite, and rinsed with THF (1.5 volumes). The filtrate is washed with water (2x5 vol) and concentrated to afford the title product as an oil.

### Example 10: Preparation of (3S,3aR,6aR)-3a-bromohexahydrofuro[2,3-b]furan-3-yl acetate

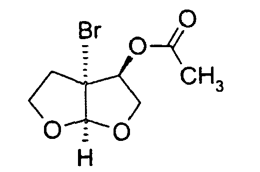

A reactor is charged with 0.1N NaHPO₄ (pH=7.0, 7.5 vol). 3a-bromohexahydrofuro [2,3-*b*] furan-3-yl acetate (leq, 1 wt) (with a pH adjustment using 15% NaOH if necessary) is added and the solution heated to 35 +/- 3 °C. An appropriate esterase is added and the pH is kept between 6.8 and 7.2 with the addition of 15% NaOH when necessary. The reaction is followed by chiral GC until all of the undesired acetate has been hydrolyzed (ca. 18 h). Celite (0.5 wt) is added, followed by methylene chloride (4.0 vol) and the slurry stirred for 15 5 min. Filtration through celite with a methylene chloride (2.0 vol) wash gives a biphasic mixture that is separated. The organic is washed with water (3 x 1 vol) to remove the undesired alcohol, and then with 10% NaCl (2 vol). The organic layer is concentrated to an oil.

### Example 11: Preparation of (3S,3aR,6aR)-3a-bromohexahydrofuro[2,3-b]furan-3-yl acetate

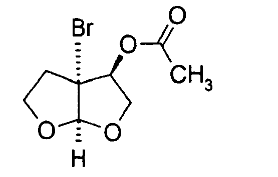

A reactor was charged with 0.1N NaHPO₄ (pH=7.0, 7.5 vol) and 3a-bromohexahydrofuro[2,3-*b*]furan-3-yl acetate from the first chemical conversion in Example 9 (1eq, 1 wt) (with a pH adjustment using 15% NaOH) was added and the solution heated to 35 +/- 3 °C. ChiroCLEC-PC (<0.01 wt.) was added and the pH was kept between 6.8 and 7.2 with the addition of 15% NaOH when necessary. The reaction was followed by chiral GC until all of the undesired acetate was hydrolyzed (ca. 18 h). (Only one enantiomer of the minor diastereomer is deacetylated.) The enzyme was filtered off and the desired acetate [(3*S*,3a*R*,6a*R*)-3a-bromohexahydrofuro[2,3-*b*]furan-3-yl acetate, ~3:1 diastereomeric ratio] was extracted into heptane which was then concentrated to an oil. This procedure may be applied with equal success to the resolution of a 95:5 diastereomeric ratio of the starting material.

### Example 12: Preparation of (3S,3aR,6aR)-3a-bromohexahydrofuro[2,3-b]furan-3-ol

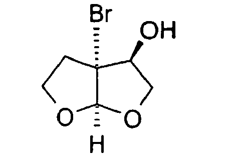

A reactor was charged with 3a-bromohexahydrofuro[2,3-*b*]furan-3-ol from Example 3 (leq, 1 wt), vinyl acetate (2.3 equiv., 1.0 vol.), toluene (5 vol.), and ChiroCLEC-PC (<0.01 wt.). The reaction was followed by chiral GC until all of the undesired acetate was hydrolyzed (ca. 3 h). (Only one enantiomer of the minor diastereomer is acetylated.) The reaction mixture was filtered and the desired alcohol [(3*S*,3a*R*,6a*R*)-3a-bromohexahydrofuro[2,3-*b*]furan-3-ol] was extracted into water. Residual undesired acetate [(3*R*,3a*S*,6a*S*)-3a-bromohexahydrofuro[2,3-*b*]furan-3-yl acetate] was removed from the aqueous mixture by extraction with heptane. The desired alcohol was then extracted into tert-butyl methyl ether and the major diastereomer was enriched to 95:5 by successive washes with water. The organic layer was concentrated to an oil that solidifies on standing. This procedure may be applied with equal success to the resolution of a 95:5 diastereomeric ratio of the starting material without the necessity for extractive purification.

### Example 13: Preparation of rel-(3S,3aR,6aR)-3a-bromohexahydrofuro[2,3-b]furan-3-ol

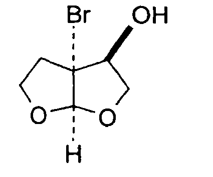

The solution of (+/-)-1-(4,5-dihydrofuran-3-yl)ethane-1,2-diol from Example 2 was cooled to ~-10°C and titrated with NBS (~ 0.69 eq based on ethyl 4,5-dihydrofuran-3-yl(oxo)acetate used in example 2, ~ 0.94 wt.), The titration was accomplished by monitoring the exotherm associated with the portionwise addition of NBS carried out over ~1 h at ~-10 °C. The reaction mixture was stirred for another ~30 min and was treated with 10 % sodium sulfite solution (6 vol.). The resulting solution (a 3:1 diastereomeric mixture) was concentrated to about one half the original volume in vacuo at 35 - 40 °C and extracted with methyl tert-butyl ether (5 x 5 vol.). The combined organics were washed with water (5 x 5 vol.), and concentrated to an oil to afford 3a-bromohexahydrofuro[2,3-b]furan-3-ol as a diastereomeric mixture of 95 to 5 (~40% yield from ethyl 4,5-dihydrofuran-3-yl(oxo)acetate. The washes were combined and extracted with ethyl acetate (2x10 vol). The extracts, made up of a 3 : 1 mixture of diastereomers, were cycled through the above extraction process to isolate ~10% additional product as a 95 : 5 diastereomeric mixture. Overall yield is ~50% (two steps).

### Example 14: Preparation of hexahydrofuro[2,3-b]furan-3-yl acetate

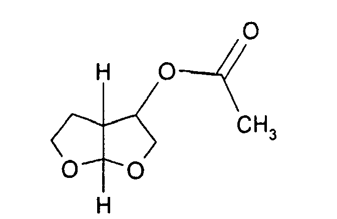

3a-bromohexahydrofuro[2,3-b]furan-3-ol (1 wt., 1 eq) (3:1 mixture of diastereomers unresolved), THF (4.2 wt.), and triethylamine (0.58 wt, 1.2 eq.) were charged to a reactor followed by a slurry of palladium on carbon (0.28 wt, 5% Pd/C, 50% water) in water (0.86 wt.). The mixture was subjected to H₂ for ~8 hours and the catalyst was removed by filtration and washed with THF (2x1 wt.). The resulting solution was concentrated to ~half the volume and successively charged with ethyl acetate and concentrated to ~half the volume to reduce water levels. Dichloromethane was charged (6.5 wt.) followed by triethylamine (0.58 wt., 1.2 eq.), and DMAP (0.005 wt., 0.01 eq.) and the mixture was cooled to ~5°C. Acetic anhydride (0.58 wt., 1.2 eq.), was added over 30 min while keeping the temperature at 5-10 °C. Reaction mixture was warmed to ~23°C over 1.5 h at which point the acetylation was complete. Methanol (0.076 wt., 0.50 eq.) was charged over 10 min while controlling the temperature at 23-27 °C. After an additional 30 min of stirring, the organic layer was successively washed with water (2x2.5 wt.) and 3%HCl (aq) (2.5 wt.). The organic layer was concentrated to the title compound as a free flowing oil.

### Example 15: Preparation of rel-(3R,3aS,6aR)-hexahydrofuro[2,3-b]furan-3-yl acetate

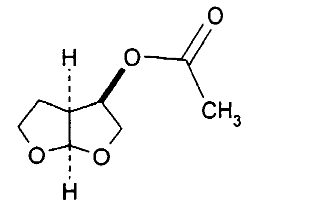

Rel-(3S,3aR,6aR)-3a-bromohexahydrofuro[2,3-b]furan-3-ol (1 wt., 1 eq) (95:5 mixture of diastereomers, unresolved), THF (4.2 wt.), and triethylamine (0.58 wt, 1.2 eq.) were charged to a reactor followed by a slurry of palladium on carbon (0.28 wt, 5% Pd/C, 50% water) in water (0.86 wt.). The mixture was subjected to H₂ for ~8 hours and the catalyst was removed by filtration and washed with THF (2x1 wt.). The resulting solution was concentrated to ~half the volume and successively charged with ethyl acetate and concentrated to ~half the volume to reduce water levels. Dichloromethane was charged (6.5 wt.) followed by triethylamine (0.58 wt., 1.2 eq.), and DMAP (0.005 wt., 0.01 eq.) and the mixture was cooled to ~5 °C. Acetic anhydride (0.58 wt., 1.2 eq.), was added over 30 min while keeping the temperature at 5-10 °C. Reaction mixture was warmed to ~23 °C over 1.5 h at which point the acetylation was complete. Methanol (0.076 wt., 0.50 eq.) was charged over 10 min while controlling the temperature at 23-27 °C. After an additional 30 min of stirring, the organic layer was successively washed with water (2x2.5 wt.) and 3%HCl (aq) (2.5 wt.). The organic layer was concentrated to the title compound as a free flowing oil.

### Example 16: Preparation of (3R,3aS,6aR)-hexahydrofuro[2,3-b]furan-3-ol

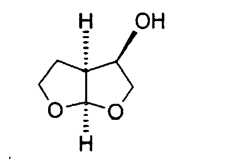

(3S,3aR,6aR)-3a-bromohexahydrofuro[2,3-b]furan-3-ol (as a 95 : 5 diastereomeric ratio resolved) (1 eq., 1 wt.), THF (4.2 wt.), and triethylamine (0.58 wt, 1.2 eq.) were charged to a reactor followed by a slurry of palladium on carbon (0.28 wt, 5% Pd/C, 50% water) in water (0.86 wt.). The mixture was subjected to H₂ for ~8 hours and the catalyst was removed by filtration and washed with THF (2x1 wt.). The resulting solution was concentrated to a water-wet oil which was diluted in ethyl acetate (5 wt.) and concentrated to the title compound as a free flowing oil.

## Claims

1. A process for the preparation of compounds of formula (I) 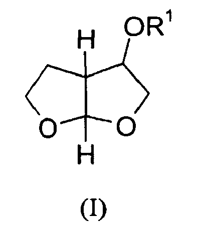 diastereoisomers, enantiomers, and mixtures thereof,
wherein R¹ is hydrogen, comprising:
a) treating a compound of formula (XII) 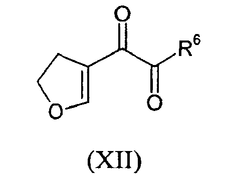 wherein:
R⁶ is halogen, -OR⁷, or -NR⁸R⁹;
R⁷ is hydrogen, C₁₋₆alkyl, C₃₋₈cycloalkyl, C₆₋₁₄aryl, or C₆₋₁₄arylC₁₋₆alkyl;
R⁸ and R⁹ are independently selected from hydrogen, C₁₋₆alkyl, C₃₋₈cycloalkyl, C₆₋₁₄aryl, and C₆₋₁₄arylC₁₋₆alkyl;
with a first reducing agent to form an alcohol of formula (III) 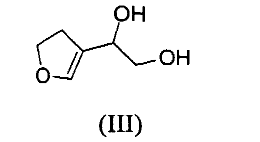
b) treating the alcohol with bromine, iodine, iodine monochloride, an N-fluoro bis sulfonamide or a compound of formula (XIII) 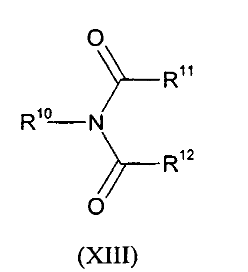 wherein:
R¹⁰ is chlorine, bromine, or iodine; and
R¹¹ and R¹² are independently selected from C₁₋₆alkyl, C₃₋₈cycloalkyl, C₆₋₁₄aryl, and C₆₋₁₄arylC₁₋₆alkyl, or R¹¹ and R¹² together with the atoms to which they are attached form a 5-8 membered ring;
to form a compound of formula (II) 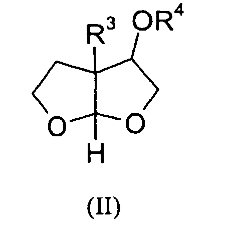 wherein R³ is halogen, and R⁴ is hydrogen; and
c) treating a compound of formula (II) with a second reducing agent to afford a compound of formula (I), wherein R¹ is hydrogen.

2. A process for the preparation of compounds of formula (I) according to claim 1, wherein said first reducing agent is selected from the group consisting of di-*iso*butylaluminum hydride (DIBAL), sodium borohydride, and lithium aluminum hydride, R⁶ in the compound of formula (XII) is -OR⁷ wherein R⁷ is C₁₋₆alkyl, the compound of formula (XIII) is N-bromosuccinimide, and the second reducing agent is palladium on carbon in combination with hydrogen.

3. A process for the preparation of compounds of formula (I) 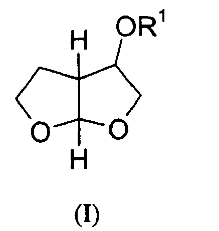 diastereoisomers, enantiomers, and mixtures thereof,
wherein R¹ is -C(O)R²; and R² is C₁₋₆alkyl, C₃₋₈cycloalkyl, C₆₋₁₄aryl, or C₆₋₁₄arylC₁₋₆alkyl, comprising:
a) treating a compound of formula (XII) 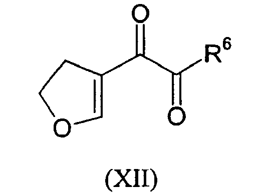 wherein:
R⁶ is halogen, -OR⁷, or -NR⁸R⁹;
R⁷ is hydrogen, C₁₋₆alkyl, C₃₋₈cycloalkyl, C₆₋₁₄aryl, or C₆₋₁₄arylC₁₋₆alkyl; and
R⁸ and R⁹ are independently selected from hydrogen, C₁₋₆alkyl, C₃₋₈cycloalkyl, C₆₋₁₄aryl, and C₆₋₁₄arylC₁₋₆alkyl;
with a first reducing agent to form an alcohol of formula (III) 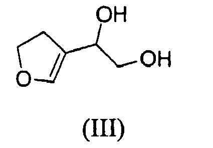
b) treating the alcohol with bromine, iodine, iodine monochloride, an N-fluoro bis sulfonamide or a compound of formula (XIII) 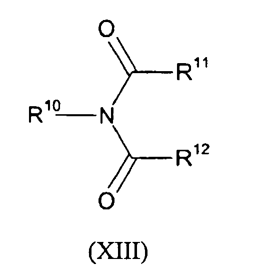 wherein:
R¹⁰ is chlorine, bromine, or iodine; and
R¹¹ and R¹² are independently selected from C₁₋₆alkyl, C₃₋₈cycloalkyl, C₆₋₁₄aryl, and C₆₋₁₄arylC₁₋₆alkyl, or R¹¹ and R¹² together with the atoms to which they are attached form a 5-8 membered ring;
to form a compound of formula (II) 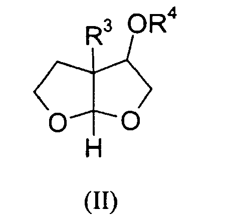 wherein R³ is halogen, and R⁴ is hydrogen;
c) treating a compound of formula (II) with a second reducing agent to afford a compound of formula (I), wherein R¹ is hydrogen; and
d) resolving to form a compound of formula (I), wherein R¹ is -C(O)R² and R² is C₁₋₆alkyl, C₃₋₈cycloalkyl, C₆₋₁₄aryl, or C₆₋₁₄arylC₁₋₆alkyl.

4. A process for the preparation of compounds of formula (I) 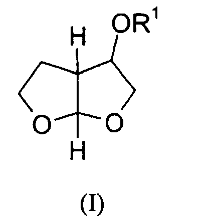 diastereoisomers, enantiomers, and mixtures thereof,
wherein R¹ is hydrogen or -C(O)R² wherein R² is C₁₋₆alkyl, C₃₋₈cycloalkyl, C₆₋₁₄aryl, or C₆₋₁₄arylC₁₋₆alkyl,
comprising reducing a compound of formula (XII) 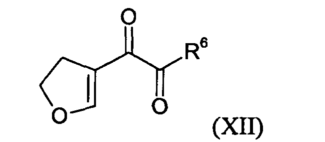 wherein R⁶ is halogen, -OR⁷, or -NR⁸R⁹;
R⁷ is hydrogen, C₁₋₆alkyl, C₃₋₈cycloalkyl, C₆₋₁₄aryl, or C₆₋₁₄arylC₁₋₆alkyl; and
R⁸ and R⁹ are independently selected from hydrogen, C₁₋₆alkyl, C₃₋₈cycloalkyl, C₆₋₁₄aryl, and C₆₋₁₄arylC₁₋₆alkyl; to afford an alcohol of formula III 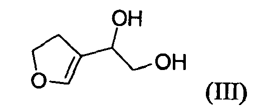 and reacting a compound of formula (III) with a reagent capable of providing a proton source to yield a compound of formula (I).

5. A process for the preparation of compounds of formula (I) 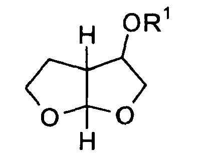 wherein R¹ is hydrogen,
comprising treating a compound of formula (III) 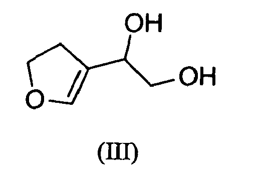 with an acid selected from the group consisting of hydrochloric acid, hydrobromic acid, hydroiodic acid, acetic acid, sulfuric acid, and sulfonic acid.

6. A process for the preparation of a compound of formula (V) 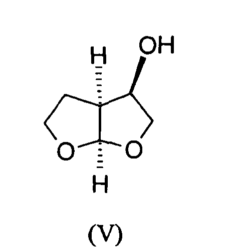 containing from about 0 to 10% of other diastereoisomers, comprising:
a) treating a compound of formula (XII) 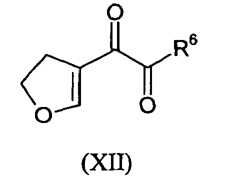 wherein:
R⁶ is halogen, -OR⁷, or -NR⁸R⁹;
R⁷ is hydrogen, C₁₋₆alkyl, C₃₋₈cycloalkyl, C₆₋₁₄aryl, or C₆₋₁₄arylC₁₋₆alkyl; and
R⁸ and R⁹ are independently selected from hydrogen, C₁₋₆alkyl, C₃₋₈cycloalkyl, C₆₋₁₄aryl, and C₆₋₁₄arylC₁₋₆alkyl;
with a first reducing agent to form an alcohol of formula (III) 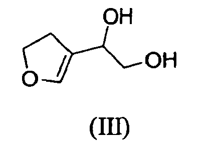
b) treating the alcohol with bromine, iodine, iodine monochloride, an N-fluoro bis sulfonamide or a compound of formula (XIII) 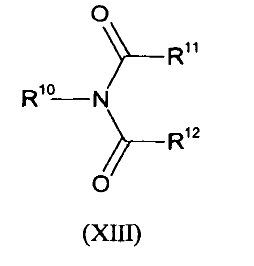 wherein:
R¹⁰ is chlorine, bromine, or iodine; and
R¹¹ and R¹² are independently selected from C₁₋₆alkyl, C₃₋₈cycloalkyl, C₆₋₁₄aryl, and C₆₋₁₄arylC₁₋₆alkyl, or R¹¹ and R¹² together with the atoms to which they are attached form a 5-8 membered ring;
to form a compound of formula (II) 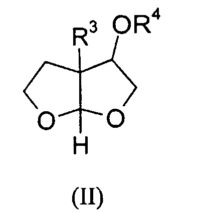 wherein R³ is halogen and R⁴ is hydrogen;
c) treating a compound of formula (II) with a second reducing agent to afford a compound of formula (I) 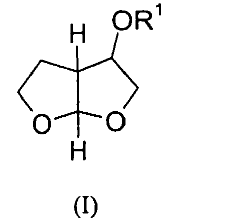 wherein R¹ is hydrogen; and
d) resolving to form a compound of formula (I), wherein R¹ is hydrogen or -C(O)R² and R² is C₁₋₆alkyl, C₃₋₈cycloalkyl, C₆₋₁₄aryl, or C₆₋₁₄arylC₁₋₆alkyl.

7. A compound of formula (II) 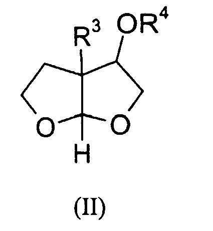 wherein:
R³ is halogen;
R⁴ is hydrogen or -C(O)R⁵;
R⁵ is hydrogen, C₁₋₆alkyl, C₃₋₈cycloalkyl, C₆₋₁₄aryl, or C₆₋₁₄arylC₁₋₆alkyl; and
diastereoisomers, enantiomers, and mixtures thereof.

8. A compound of formula (II) according to claim 7 wherein R³ is bromine and R⁴ is hydrogen.

9. A compound of formula (II) according to claim 7 wherein R³ is bromine, R⁴ is -C(O)R⁵ and R⁵ is C₁₋₆alkyl,

10. A compound of formula (II) according to claim 7 wherein R³ is bromine, R⁴ is -C(O)R⁵, and R⁵ is -CH₃.

11. A process for the preparation of compounds of formula (II) 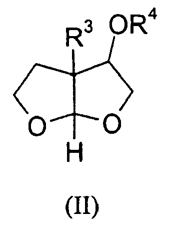 wherein:
R³ is halogen;
R⁴ is hydrogen or -C(O)R⁵ ;
R⁵ is hydrogen, C₁₋₆alkyl, C₃₋₈cycloalkyl, C₆₋₁₄aryl, or C₆₋₁₄arylC₁₋₆alkyl; and
diastereoisomers, enantiomers, and mixtures thereof, comprising:
a) treating a compound of formula (XII) 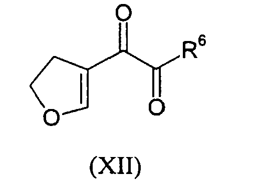 wherein:
R⁶ is halogen, -OR⁷, or -NR⁸R⁹;
R⁷ is hydrogen, C₁₋₆alkyl, C₃₋₈cycloalkyl, C₆₋₁₄aryl, or C₆₋₁₄arylC₁₋₆alkyl; and
R⁸ and R⁹ are independently selected from hydrogen, C₁₋₆alkyl, C₃₋₈cycloalkyl, C₆₋₁₄aryl, and C₆₋₁₄arylC₁₋₆alkyl;
with a reducing agent to form an alcohol of formula (III) 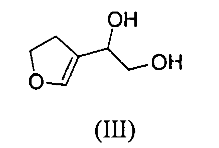
b) treating said alcohol with bromine, iodine, iodine monochloride, an N-fluoro bis sulfonamide or a compound of formula (XIII) 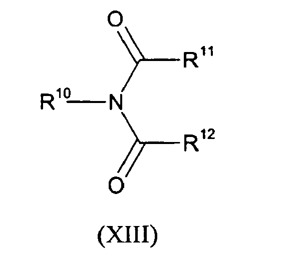 wherein:
R¹⁰ is chlorine, bromine, or iodine; and
R¹¹ and R¹² are independently selected from C₁₋₆alkyl, C₃₋₈cycloalkyl, C₆₋₁₄aryl, and C₆₋₁₄arylC₁₋₆alkyl, or R¹¹ and R¹² together with the atoms to which they are attached form a 5-8 membered ring; to form a compound of formula (II), wherein R³ is halogen and R⁴ is hydrogen; and
c) resolving to yield a compound of formula (II) wherein R³ is halogen; R⁴ is hydrogen or - C(O)R⁵; and R⁵ is hydrogen, C₁₋₆alkyl, C₃₋₈cycloalkyl, C₆₋₁₄aryl, or C₆₋₁₄arylC₁₋₆alkyl.

12. A compound of formula (XIX)

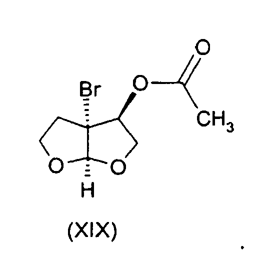

13. A process for the preparation of a compound of formula (XIX) 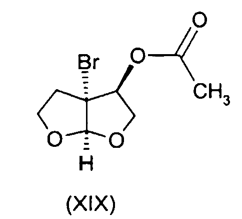 comprising:
a) treating a compound of formula (XII) 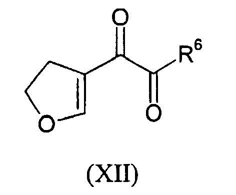 wherein:
R⁶ is halogen, -OR⁷, or -NR⁸R⁹;
R⁷ is hydrogen, C₁₋₆alkyl, C₃₋₈cycloalkyl, C₆₋₁₄aryl, or C₆₋₁₄arylC₁₋₆alkyl; and
R⁸ and R⁹ are independently selected from hydrogen, C₁₋₆alkyl, C₃₋₈cycloalkyl, C₆₋₁₄aryl, and C₆₋₁₄arylC₁₋₆alkyl;
with a reducing agent to form an alcohol of formula (III) 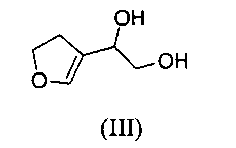
b) treating said alcohol with bromine, iodine, iodine monochloride, an N-fluoro bis sulfonamide or a compound of formula (XIII) 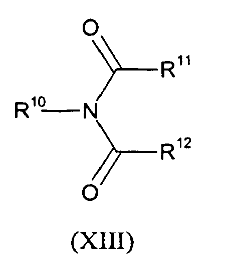 wherein:
R¹⁰ is chlorine, bromine, or iodine; and
R¹¹ and R¹² are independently selected from C₁₋₆alkyl, C₃₋₈cycloalkyl, C₆₋₁₄aryl, and C₆₋₁₄arylC₁₋₆alkyl, or R¹¹ and R¹² together with the atoms to which they are attached form a 5-8 membered ring; and
c) optionally resolving to yield a compound of formula (XIX).

14. A compound of formula (XX)

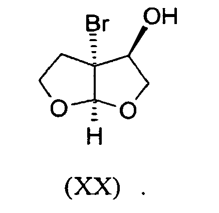

15. A process for the preparation of a compound of formula (XX) 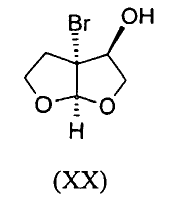 comprising:
a) treating a compound of formula (XII) 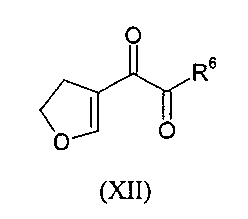 wherein:
R⁶ is halogen, -OR⁷, or NR⁸R⁹;
R⁷ is hydrogen, C₁₋₆alkyl, C₃₋₈cycloalkyl, C₆₋₁₄aryl, or C₆₋₁₄arylC₁₋₆alkyl; and
R⁸ and R⁹ are independently selected from hydrogen, C₁₋₆alkyl, C₃₋₈cycloalkyl, C₆₋₁₄aryl, and C₆₋₁₄arylC₁₋₆alkyl;
with a reducing agent to form an alcohol of formula (III) 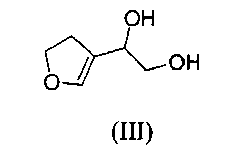
b) treating said alcohol with N-bromosuccinimide to form a compound of formula (XX); and
c) optionally resolving to yield diastereoisomers of compounds of formula (XX).

16. A compound of formula (III)

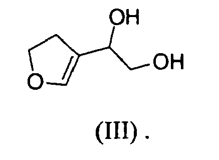

17. A process for the preparation of compound (III) 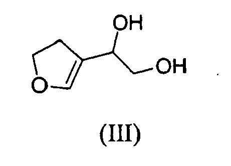 comprising treating a compound of formula (XII) 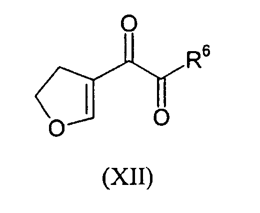 wherein R⁶ is halogen, -OR⁷, or -NR⁸R⁹; where R⁷ is hydrogen, C₁₋₆alkyl, C₃₋₈cycloalkyl, C₆₋₁₄aryl, or C₆₋₁₄arylC₁₋₆alkyl; and R⁸ and R⁹ are independently selected from hydrogen, C₁₋₆alkyl, C₃₋₈cycloalkyl, C₆₋₁₄aryl, and C₆₋₁₄arylC₁₋₆alkyl; with a reducing agent.

18. A process according to claim 18 wherein the reducing agent is selected from the group consisting of di-isobutylaluminium hydride (DIBAL), sodium borohydride, and lithium aluminum hydride.

19. A process for the preparation of compounds of formula I, II, V, XIV, XIX, and XX, according to any of claims 1, 3, 4, 6, 11, 13, or 15 wherein the first reducing agent is selected from the group consisting of di-*iso*butylaluminium hydride (DIBAL), sodium borohydride, and lithium aluminum hydride, wherein R⁶ in the compound of formula (XII) is -OR⁷ where R⁷ is C₁₋₆alkyl, wherein the compound of formula (XIII) is N-bromosuccinimide, and the second reducing agent is palladium on carbon in combination with hydrogen.

20. A process according to any of claims 1, 2, 3, or 4 further comprising the step of resolving to obtain single enantiomers.

## Patentansprüche

1. Verfahren zur Herstellung von Verbindungen der Formel (I), 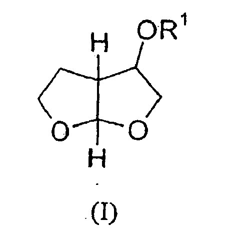 Diastereoisomeren, Enantiomeren und Gemischen davon,
wobei R¹ Wasserstoff ist, umfassend:
a) Behandeln einer Verbindung der Formel (XII) 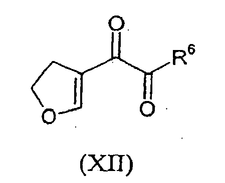 wobei:
R⁶ Halogen, -OR⁷ oder -NR⁸R⁹ ist;
R⁷ Wasserstoff, C₁₋₆-Alkyl, C₃₋₈-Cycloalkyl, C₆₋₁₄-Aryl oder C₆₋₁₄-Aryl-C₁₋₆-alkyl ist;
R⁸ und R⁹ unabhängig aus Wasserstoff, C₁₋₆-Alkyl, C₃₋₈-Cycloalkyl, C₆₋₁₄-Aryl und C₆₋₁₄-Aryl-C₁₋₆-alkyl ausgewählt sind;
mit einem ersten Reduktionsmittel unter Bildung eines Alkohols der Formel (III) 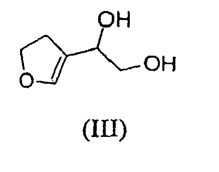
b) Behandeln des Alkohols mit Brom, Iod, Iodmonochlorid, einem N-Fluorbissulfonamid oder einer Verbindung der Formel (XIII) 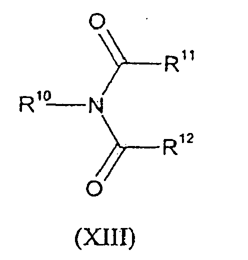 wobei:
R¹⁰ Chlor, Brom oder Iod ist; und
R¹¹ und R¹² unabhängig aus C₁₋₆-Alkyl, C₃₋₈-Cycloalkyl, C₆₋₁₄-Aryl und C₆₋₁₄-Aryl-C₁₋₆-alkyl ausgewählt sind oder R¹¹ und R¹² zusammen mit den Atomen, an die sie gebunden sind, einen 5- bis 8-gliedrigen Ring bilden;
unter Bildung einer Verbindung der Formel (II) 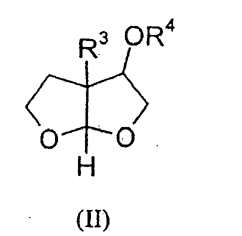 wobei R³ Halogen ist und R⁴ Wasserstoff ist; und
c) Behandeln einer Verbindung der Formel (II) mit einem zweiten Reduktionsmittel, um eine Verbindung der Formel (I) zu liefern, wobei R¹ Wasserstoff ist.

2. Verfahren zur Herstellung von Verbindungen der Formel (I) nach Anspruch 1, wobei das erste Reduktionsmittel ausgewählt ist aus Diisobutylaluminiumhydrid (DIBAL), Natriumborhydrid und Lithiumaluminiumhydrid, R⁶ in der Verbindung der Formel (XII) -OR⁷ ist, wobei R⁷ C₁₋₆-Alkyl ist, die Verbindung der Formel (XIII) N-Bromsuccinimid ist und das zweite Reduktionsmittel Palladium auf Kohlenstoff in Verbindung mit Wasserstoff ist.

3. Verfahren zur Herstellung von Verbindungen der Formel (I), 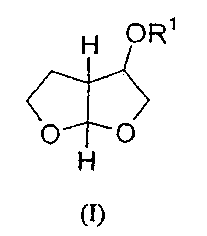 Diastereoisomeren, Enantiomeren und Gemischen davon,
wobei R¹ -C(O)R² ist; und R² C₁₋₆-Alkyl, C₃₋₈-Cycloalkyl, C₆₋₁₄-Aryl oder C₆₋₁₄-Aryl-C₁₋₆-alkyl ist, umfassend:
a) Behandeln einer Verbindung der Formel (XII) 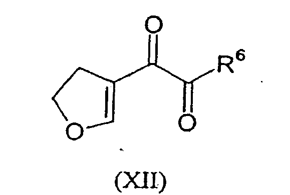 wobei:
R⁶ Halogen, -OR⁷ oder -NR⁸R⁹ ist;
R⁷ Wasserstoff, C₁₋₆-Alkyl, C₃₋₈-Cycloalkyl, C₆₋₁₄-Aryl oder C₆₋₁₄-Aryl-C₁₋₆-alkyl ist; und
R⁸ und R⁹ unabhängig aus Wasserstoff, C₁₋₆-Alkyl, C₃₋₈-Cycloalkyl, C₆₋₁₄-Aryl und
C₆₋₁₄-Aryl-C₁₋₆-alkyl ausgewählt sind;
mit einem ersten Reduktionsmittel unter Bildung eines Alkohols der Formel (III) 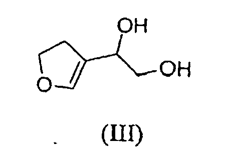
b) Behandeln des Alkohols mit Brom, Iod, Iodmonochlorid, einem N-Fluorbissulfonamid oder einer Verbindung der Formel (XIII) 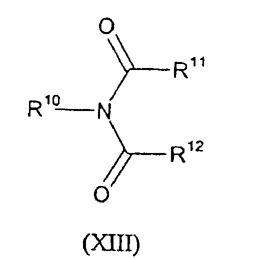 wobei:
R¹⁰ Chlor, Brom oder Iod ist; und
R¹¹ und R¹² unabhängig ausgewählt sind aus C₁₋₆-Alkyl, C₃₋₈-Cycloalkyl, C₆₋₁₄-Aryl und C₆₋₁₄-Aryl-C₁₋₆-alkyl oder R¹¹ und R¹² zusammen mit den Atomen, an die sie gebunden sind, einen 5- bis 8-gliedrigen Ring bilden;
unter Bildung einer Verbindung der Formel (II) 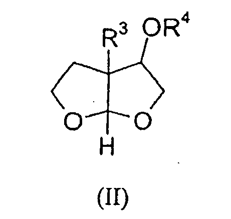 wobei R³ Halogen ist und R⁴ Wasserstoff ist;
c) Behandeln einer Verbindung der Formel (II) mit einem zweiten Reduktionsmittel, um eine Verbindung der Formel (I) zu liefern, wobei R¹ Wasserstoff ist; und
d) Lösen unter Bildung einer Verbindung der Formel (I), wobei R¹ -C(O)R² ist und R² C₁₋₆-Alkyl, C₃₋₈-Cycloalkyl, C₆₋₁₄-Aryl oder C₆₋₁₄-Aryl-C₁₋₆-alkyl ist.

4. Verfahren zur Herstellung von Verbindungen der Formel (I), 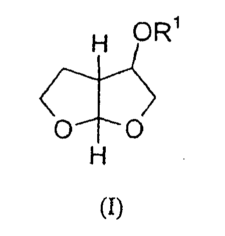 Diastereoisomeren, Enantiomeren und Gemischen davon,
wobei R¹ Wasserstoff oder -C(O)R² ist, wobei R² C₁₋₆-Alkyl, C₃₋₈-Cycloalkyl, C₆₋₁₄-Aryl oder C₆₋₁₄Aryl-C₁₋₆-alkyl ist;
umfassend die Reduktion einer Verbindung der Formel (XII) 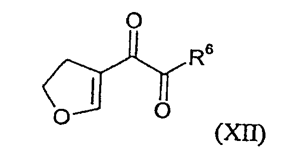 wobei R⁶ Halogen, -OR⁷ oder -NR⁸R⁹ ist;
R⁷ Wasserstoff, C₁₋₆-Alkyl, C₃₋₈-Cycloalkyl, C₆₋₁₄-Aryl oder C₆₋₁₄-Aryl-C₁₋₆-alkyl ist; und
R⁸ und R⁹ unabhängig aus Wasserstoff, C₁₋₆-Alkyl, C₃₋₈-Cycloalkyl, C₆₋₁₄-Aryl und C₆₋₁₄-Aryl-C₁₋₆-alkyl ausgewählt sind; um einen Alkohol der Formel (III) zu liefern 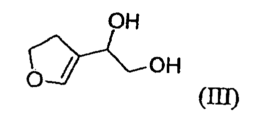 und Umsetzen einer Verbindung der Formel (III) mit einem Reagens, das zur Bereitstellung einer Protonenquelle in der Lage ist, um eine Verbindung der Formel (I) zu liefern.

5. Verfahren zur Herstellung von Verbindungen der Formel (I) 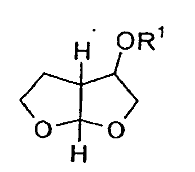 wobei R¹ Wasserstoff ist,
umfassend das Behandeln einer Verbindung der Formel (III) 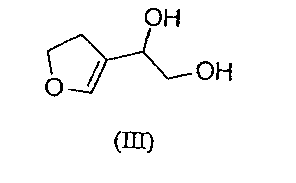 mit einer Säure, ausgewählt aus Chlorwasserstoffsäure, Bromwasserstoffsäure, Iodwasserstoffsäure, Essigsäure, Schwefelsäure und Sulfonsäure.

6. Verfahren zur Herstellung einer Verbindung der Formel (V) 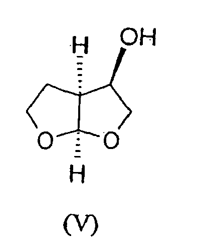 welche etwa 0 bis 10% andere Diastereoisomere enthält, umfassend:
a) Behandeln einer Verbindung der Formel (XII) 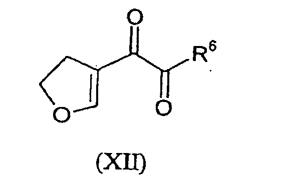 wobei:
R⁶ Halogen, -OR⁷ oder -NR⁸R⁹ ist;
R⁷ Wasserstoff, C₁₋₆-Alkyl, C₃₋₈-Cycloalkyl, C₆₋₁₄-Aryl oder C₆₋₁₄-Aryl-C₁₋₆-alkyl ist; und
R⁸ und R⁹ unabhängig ausgewählt sind aus Wasserstoff, C₁₋₆-Alkyl, C₃₋₈₋Cycloalkyl, C₆₋₁₄-Aryl und C₆₋₁₄-Aryl-C₁₋₆-alkyl;
mit einem ersten Reduktionsmittel unter Bildung eines Alkohols der Formel (III) 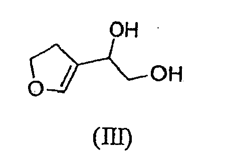
b) Behandeln des Alkohols mit Brom, Iod, Iodmonochlorid, einem N-Fluorbissulfonamid oder einer Verbindung der Formel (XIII) 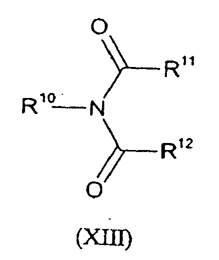 wobei:
R¹⁰ Chlor, Brom oder Iod ist; und
R¹¹ und R¹² unabhängig aus C₁₋₆-Alkyl, C₃₋₈-Cycloalkyl, C₆₋₁₄-Aryl und C₆₋₁₄-Aryl-C₁₋₆-alkyl ausgewählt sind oder R¹¹ und R¹² zusammen mit den Atomen, an die sie gebunden sind, einen 5- bis 8-gliedrigen Ring bilden;
unter Bildung einer Verbindung der Formel (II) 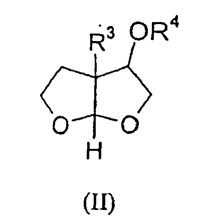 wobei R³ Halogen ist und R⁴ Wasserstoff ist,
c) Behandeln einer Verbindung der Formel (II) mit einem zweiten Reduktionsmittel, um eine Verbindung der Formel (I) zu liefern 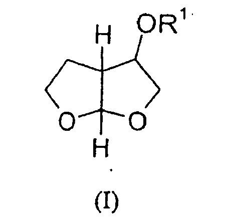 wobei R¹ Wasserstoff ist; und
d) Lösen unter Bildung einer Verbindung der Formel (I), wobei R¹ Wasserstoff oder -C(O)R² ist und R² C₁₋₆-Alkyl, C₃₋₈-Cycloalkyl, C₆₋₁₄-Aryl oder C₆₋₁₄-Aryl-C₁₋₆₋alkyl ist.

7. Verbindung der Formel (II) 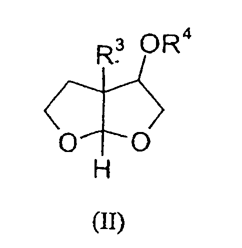 wobei:
R³ Halogen ist;
R⁴ Wasserstoff oder -C(O)R⁵ ist;
R⁵ Wasserstoff, C₁₋₆-Alkyl, C₃₋₈-Cycloalkyl, C₆₋₁₄-Aryl oder C₆₋₁₄-Aryl-C₁₋₆-alkyl ist; und
Diastereoisomere, Enantiomere und Gemische davon.

8. Verbindung der Formel (II) nach Anspruch 7, wobei R³ Brom ist und R⁴ Wasserstoff ist.

9. Verbindung der Formel (II) nach Anspruch 7, wobei R³ Brom ist, R⁴ -C(O)R⁵ ist und R⁵ C₁₋₆-Alkyl ist.

10. Verbindung der Formel (II) nach Anspruch 7, wobei R³ Brom ist, R⁴ -C(O)R⁵ ist und R⁵ -CH₃ ist.

11. Verfahren zur Herstellung von Verbindungen der Formel (II) 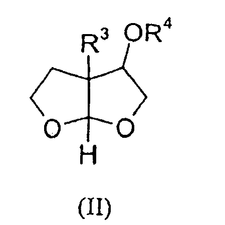 wobei:
R³ Halogen ist;
R⁴ Wasserstoff oder -C(O)R⁵ ist;
R⁵ Wasserstoff, C₁₋₆-Alkyl, C₃₋₈-Cycloalkyl, C₆₋₁₄-Aryl oder C₆₋₁₄-Aryl-C₁₋₆-alkyl ist; und
Diastereoisomeren, Enantiomeren und Gemischen davon, umfassend:
a) Behandeln einer Verbindung der Formel (XII) 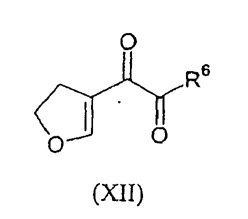 wobei:
R⁶ Halogen, -OR⁷ oder -NR⁸R⁹ ist;
R⁷ Wasserstoff, C₁₋₆-Alkyl, C₃₋₈-Cycloalkyl, C₆₋₁₄-Aryl oder C₆₋₁₄-Aryl-C₁₋₆-alkyl ist; und
R⁸ und R⁹ unabhängig aus Wasserstoff, C₁₋₆-Alkyl, C₃₋₈-Cycloalkyl, C₆₋₁₄-Aryl und C₆₋₁₄-Aryl-C₁₋₆-alkyl ausgewählt sind;
mit einem Reduktionsmittel unter Bildung eines Alkohols der Formel (III) 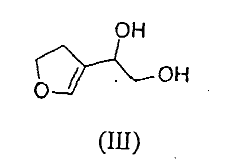
b) Behandeln des Alkohols mit Brom, Iod, Iodmonochlorid, einem N-Fluorbissulfonamid oder einer Verbindung der Formel (XIII) 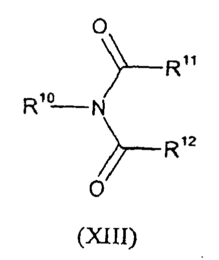 wobei:
R¹⁰ Chlor, Brom oder Iod ist; und
R¹¹ und R¹² unabhängig aus C₁₋₆-Alkyl, C₃₋₈-Cycloalkyl, C₆₋₁₄-Aryl und C₆₋₁₄-Aryl-C₁₋₆-alkyl ausgewählt sind oder R¹¹ und R¹² zusammen mit den Atomen, an die sie gebunden sind, einen 5- bis 8-gliedrigen Ring bilden; unter Bildung einer Verbindung der Formel (II), wobei R³ Halogen ist und R⁴ Wasserstoff ist; und
c) Lösen, um eine Verbindung der Formel (II) zu ergeben, wobei R³ Halogen ist; R⁴ Wasserstoff oder -C(O)R⁵ ist; und R⁵ Wasserstoff, C₁₋₆-Alkyl, C₃₋₈-Cycloalkyl, C₆₋₁₄-Aryl oder C₆₋₁₄-Aryl-C₁₋₆-alkyl ist.

12. Verbindung der Formel (XIX)

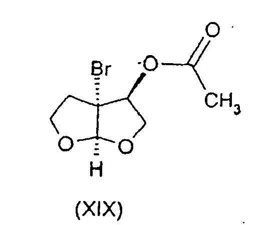

13. Verfahren zur Herstellung einer Verbindung der Formel (XIX) 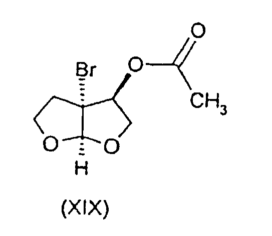 umfassend:
a) Behandeln einer Verbindung der Formel (XII) 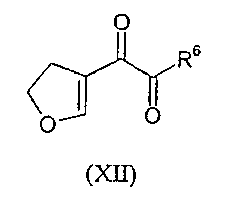 wobei:
R⁶ Halogen, -OR⁷ oder -NR⁸R⁹ ist;
R⁷ Wasserstoff, C₁₋₆-Alkyl, C₃₋₈-Cycloalkyl, C₆₋₁₄-Aryl oder C₆₋₁₄-Aryl-C₁₋₆-alkyl ist; und
R⁸ und R⁹ unabhängig aus Wasserstoff, C₁₋₆-Alkyl, C₃₋₈-Cycloalkyl, C₆₋₁₄-Aryl und C₆₋₁₄-Aryl-C₁₋₆-alkyl ausgewählt sind;
mit einem Reduktionsmittel unter Bildung eines Alkohols der Formel (III) 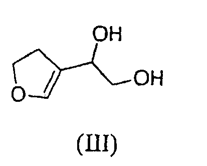
b) Behandeln des Alkohols mit Brom, Iod, Iodmonochlorid, einem N-Fluorbissulfonamid oder einer Verbindung der Formel (XIII) 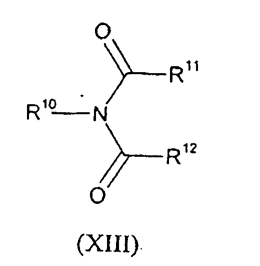 wobei:
R¹⁰ Chlor, Brom oder Iod ist; und
R¹¹ und R¹² unabhängig aus C₁₋₆-Alkyl, C₃₋₈-Cycloalkyl, C₆₋₁₄-Aryl und C₆₋₁₄-Aryl-C₁₋₆-alkyl ausgewählt sind oder R¹¹ und R¹² zusammen mit den Atomen, an die sie gebunden sind, einen 5- bis 8-gliedrigen Ring bilden; und
c) gegebenenfalls Lösen, um eine Verbindung der Formel (XIX) zu liefern.

14. Verbindung der Formel (XX)

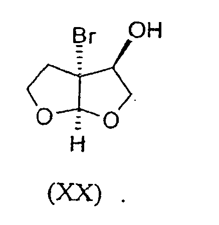

15. Verfahren zur Herstellung einer Verbindung der Formel (XX) 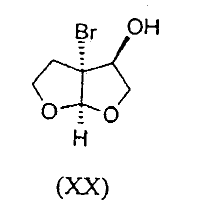 umfassend:
a) Behandeln einer Verbindung der Formel (XII) 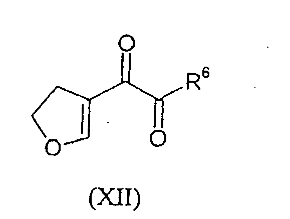 wobei:
R⁶ Halogen, -OR⁷ oder -NR⁸R⁹ ist;
R⁷ Wasserstoff, C₁₋₆-Alkyl, C₃₋₈-Cycloalkyl, C₆₋₁₄-Aryl oder C₆₋₁₄-Aryl-C₁₋₆-alkyl ist; und
R⁸ und R⁹ unabhängig aus Wasserstoff, C₁₋₆-Alkyl, C₃₋₈-Cycloalkyl, C₆₋₁₄-Aryl und C₆₋₁₄-Aryl-C₁₋₆-alkylausgewählt sind;
mit einem Reduktionsmittel unter Bildung eines Alkohols der Formel (III) 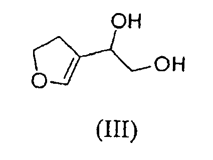
b) Behandeln des Alkohols mit N-Bromsuccinimid unter Bildung einer Verbindung der Formel (XX); und
c) gegebenenfalls Lösen, um Diastereoisomere von Verbindungen der Formel (XX) zu liefern.

16. Verbindung der Formel (III)

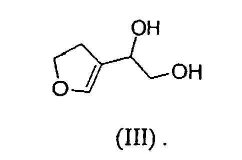

17. Verfahren zur Herstellung einer Verbindung (III) 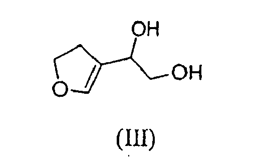 umfassend Behandeln einer Verbindung der Formel (XII) 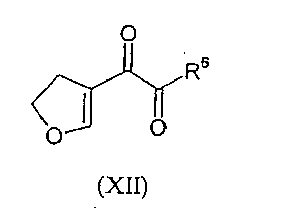 wobei R⁶ Halogen, -OR⁷ oder -NR⁸R⁹ ist; wobei R⁷ Wasserstoff, C₁₋₆-Alkyl, C₃₋₈₋Cycloalkyl, C₆₋₁₄-Aryl oder C₆₋₁₄-Aryl-C₁₋₆-alkyl ist; und R⁸ und R⁹ unabhängig ausgewählt sind aus Wasserstoff, C₁₋₆-Alkyl, C₃₋₈-Cycloalkyl, C₆₋₁₄-Aryl und C₆₋₁₄-Aryl-C₁₋₆-alkyl; mit einem Reduktionsmittel.

18. Verfahren nach Anspruch 17, wobei das Reduktionsmittel ausgewählt ist aus Diisobutylaluminiumhydrid (DIBAL), Natriumborhydrid und Lithiumaluminiumhydrid.

19. Verfahren zur Herstellung von Verbindungen der Formel I, II, V, XIV, XIX und XX nach einem der Ansprüche 1, 3, 4, 6, 11, 13 oder 15, wobei das erste Reduktionsmittel aus Diisobutylaluminiumhydrid (DIBAL), Natriumborhydrid und Lithiumaluminiumhydrid ausgewählt ist, wobei R⁶ in der Verbindung der Formel (XII) -OR⁷ ist, wobei R⁷ C₁₋₆-Alkyl ist, wobei die Verbindung der Formel (XIII) N-Bromsuccinimid ist, und das zweite Reduktionsmittel Palladium auf Kohlenstoff in Verbindung mit Wasserstoff ist.

20. Verfahren nach einem der Ansprüche 1, 2, 3 oder 4, weiter umfassend den Schritt des Lösens, um einzelne Enantiomere zu erhalten.

## Revendications

1. Procédé pour la préparation de composés de formule (I) 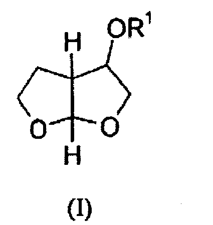 de leurs diastéréoisomères, de leurs énantiomères et de leurs mélanges,
formule dans laquelle R¹ représente l'hydrogène, comprenant :
a) le traitement d'un composé de formule (XII) 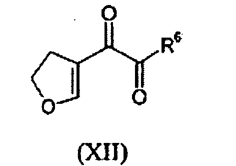 dans laquelle :
R⁶ représente un groupe halogéno, -OR⁷ ou -NR⁸R⁹ ;
R⁷ représente l'hydrogène, un groupe alkyle en C₁ à C₆, cycloalkyle en C₃ à C₈, aryle en C₆ à C₁₄ ou (aryle en C₆ à C₁₄) (alkyle en C₁ à C₆) ;
R⁸ et R⁹ sont choisis indépendamment entre l'hydrogène, des groupes alkyle en C₁ à C₆, cycloalkyle en C₃ à C₈, aryle en C₆ à C₁₄ et (aryle en C₆ à C₁₄) (alkyle en C₁ à C₆) ;
avec un premier agent réducteur pour former un alcool de formule (III) 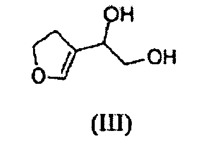
b) le traitement de l'alcool avec du brome, de l'iode, du monochlorure d'iode, un N-fluoro-bis-sulfonamide ou un composé de formule (XIII) 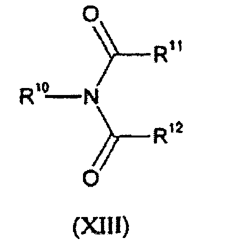 dans laquelle :
R¹⁰ représente le chlore, le brome ou l'iode ; et
R¹¹ et R¹² sont choisis indépendamment entre des groupes alkyle en C₁ à C₆, cycloalkyle en C₃ à C₈, aryle en C₆ à C₁₄ et (aryle en C₆ à C₁₄) (alkyle en C₁ à C₆), ou bien R¹¹ et R¹², conjointement avec les atomes auxquels ils sont fixés, forment un noyau penta- à octogonal ;
pour former un composé de formule (II) 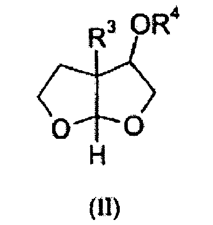 dans laquelle R³ représente un groupe halogéno et R⁴ représente l'hydrogène ; et
c) le traitement d'un composé de formule (II) avec un second agent réducteur pour obtenir un composé de formule (I), dans lequel R¹ représente l'hydrogène.

2. Procédé pour la préparation de composés de formule (I) suivant la revendication 1, dans lequel ledit premier agent réducteur est choisi dans le groupe consistant en hydrure de di-isobutylaluminium (DIBAL), borohydrure de sodium et hydrure de lithiumaluminium, R⁶ dans le composé de formule (XII) est un groupe -OR⁷ dans lequel R⁷ représente un groupe alkyle en C₁ à C₆, le composé de formule (XIII) est le N-bromosuccinimide et le second agent réducteur est le palladium sur du carbone en association avec de l'hydrogène.

3. Procédé pour la préparation de composés de formule (I) 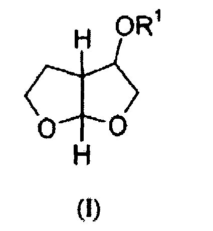 de leurs diastéréoisomères, de leurs énantiomères et de leurs mélanges,
formule dans laquelle R¹ représente un groupe -C(O)R² ; et R² représente un groupe alkyle en C₁ à C₆, cycloalkyle en C₃ à C₈, aryle en C₆ à C₁₄ ou (aryle en C₆ à C₁₄) (alkyle en C₁ à C₆), comprenant :
a) le traitement d'un composé de formule (XII) 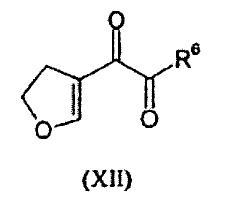 dans laquelle :
R⁶ représente un groupe halogéno, -OR⁷ ou -NR⁸R⁹ ;
R⁷ représente l'hydrogène, un groupe alkyle en C₁ à C₆, cycloalkyle en C₃ à C₈, aryle en C₆ à C₁₄ ou (aryle en C₆ à C₁₄) (alkyle en C₁ à C₆) ; et
R⁸ et R⁹ sont choisis indépendamment entre l'hydrogène, des groupes alkyle en C₁ à C₆, cycloalkyle en C₃ à C₈, aryle en C₆ à C₁₄ et (aryle en C₆ à C₁₄) (alkyle en C₁ à C₆) ;
avec un premier agent réducteur pour former un alcool de formule (III) 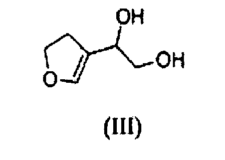
b) le traitement de l'alcool avec du brome, de l'iode, du monochlorure d'iode, un N-fluoro-bis-sulfonamide ou un composé de formule (XIII) 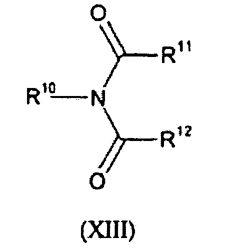 dans laquelle :
R¹⁰ représente le chlore, le brome ou l'iode ; et
R¹¹ et R¹² sont choisis indépendamment entre des groupes alkyle en C₁ à C₆, cycloalkyle en C₃ à C₈, aryle en C₆ à C₁₄ et (aryle en C₆ à C₁₄) (alkyle en C₁ à C₆), ou bien R¹¹ et R¹², conjointement avec les atomes auxquels ils sont fixés, forment un noyau penta- à octogonal ;
pour former un composé de formule (II) 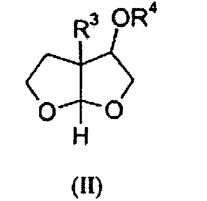 dans laquelle R³ représente un groupe halogéno et R⁴ représente l'hydrogène ;
c) le traitement d'un composé de formule (II) avec un second agent réducteur pour obtenir un composé de formule (I), dans lequel R¹ représente l'hydrogène ; et
d) une résolution pour former un composé de formule (I), dans lequel R¹ représente un groupe -C(O)R², et R² représente un groupe alkyle en C₁ à C₆, cycloalkyle en C₃ à C₈, aryle en C₆ à C₁₄ ou (aryle en C₆ à C₁₄) (alkyle en C₁ à C₆).

4. Procédé pour la préparation de composés de formule (I) 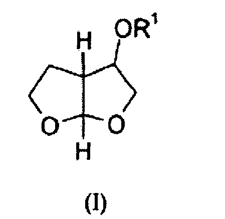 de leurs diastéréoisomères, de leurs énantiomères et de leurs mélanges,
formule dans laquelle R¹ représente l'hydrogène ou un groupe -C(O)R², dans lequel R² représente un groupe alkyle en C₁ à C₆, cycloalkyle en C₃ à C₈, aryle en C₆ à C₁₄ ou (aryle en C₆ à C₁₄) (alkyle en C₁ à C₆), comprenant :
la réduction d'un composé de formule (XII) 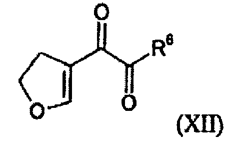 dans laquelle :
R⁶ représente un groupe halogéno, -OR⁷ ou -NR⁸R⁹ ;
R⁷ représente l'hydrogène, un groupe alkyle en C₁ à C₆, cycloalkyle en C₃ à C₈, aryle en C₆ à C₁₄ ou (aryle en C₆ à C₁₄) (alkyle en C₁ à C₆) ; et
R⁸ et R⁹ sont choisis indépendamment entre l'hydrogène, des groupes alkyle en C₁ à C₆, cycloalkyle en C₃ à C₈, aryle en C₆ à C₁₄ et (aryle en C₆ à C₁₄) (alkyle en C₁ à C₆) ; pour obtenir un alcool de formule (III) 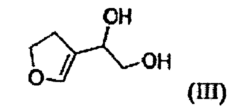
et la réaction d'un composé de formule (III) avec un réactif capable de fournir une source de protons pour obtenir un composé de formule (I).

5. Procédé pour la préparation des composés de formule (I) 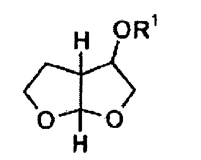 dans laquelle R¹ représente l'hydrogène,
comprenant le traitement d'un composé de formule (III) 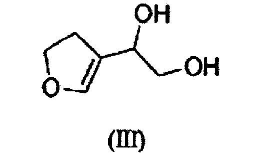 avec un acide choisi dans le groupe consistant en l'acide chlorhydrique, l'acide bromhydrique, l'acide iodhydrique, l'acide acétique, l'acide sulfurique et un acide sulfonique.

6. Procédé pour la préparation d'un composé de formule (V) 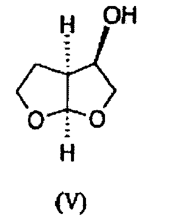 contenant environ 0 à 10 % d'autres diastéréoisomères, comprenant :
a) le traitement d'un composé de formule (XII) 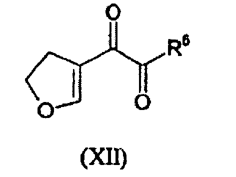 dans laquelle :
R⁶ représente un groupe halogéno, -OR⁷ ou -NR⁸R⁹ ;
R⁷ représente l'hydrogène, un groupe alkyle en C₁ à C₆, cycloalkyle en C₃ à C₈, aryle en C₆ à C₁₄ ou (aryle en C₆ à C₁₄) (alkyle en C₁ à C₆) ; et
_{R}⁸ et _{R}⁹ sont choisis indépendamment entre l'hydrogène, des groupes alkyle en C₁ à C₆, cycloalkyle en C₃ à C₈, aryle en C₆ à C₁₄ et (aryle en C₆ à C₁₄) (alkyle en C₁ à C₆) ;
avec un premier agent réducteur pour former un alcool de formule (III) 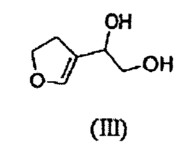
b) le traitement de l'alcool avec du brome, de l'iode, du monochlorure d'iode, un N-fluoro-bis-sulfonamide ou un composé de formule (XIII) 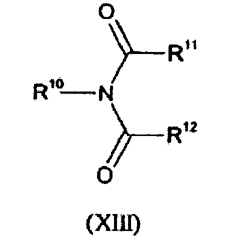 dans laquelle :
R¹⁰ représente le chlore, le brome ou l'iode ; et
R¹¹ et R¹² sont choisis indépendamment entre des groupes alkyle en C₁ à C₆, cycloalkyle en C₃ à C₈, aryle en C₆ à C₁₄ et (aryle en C₆ à C₁₄) (alkyle en C₁ à C₆), ou bien R¹¹ et R¹², conjointement avec les atomes auxquels ils sont fixés, forment un noyau penta- à octogonal ;
pour former un composé de formule (II) 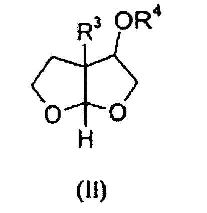 dans laquelle R³ représente un groupe halogéno et R⁴ représente l'hydrogène ;
c) le traitement d'un composé de formule (II) avec un second agent réducteur pour obtenir un composé de formule (I), 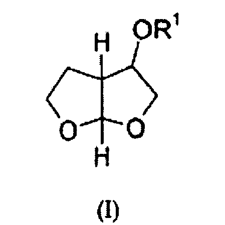 dans laquelle R¹ représente l'hydrogène ; et
d) une résolution pour former un composé de formule (I), dans lequel R¹ représente l'hydrogène ou un groupe -C (O) R², et R² représente un groupe alkyle en C₁ à C₆, cycloalkyle en C₃ à C₈, aryle en C₆ à C₁₄ ou (aryle en C₆ à C₁₄) (alkyle en C₁ à C₆) .

7. Composé de formule (II) 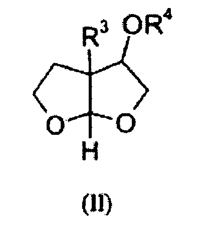 dans laquelle :
R³ représente un groupe halogéno ;
R⁴ représente l'hydrogène ou un groupe -C(O)R⁵ ;
R⁵ représente l'hydrogène, un groupe alkyle en C₁ à C₆, cycloalkyle en C₃ à C₈, aryle en C₆ à C₁₄ ou (aryle en C₆ à C₁₄) (alkyle en C₁ à C₆) ; et
ses diastéréoisomères, ses énantiomères, et leurs mélanges.

8. Composé de formule (II) suivant la revendication 7, dans lequel R³ représente le brome et R⁴ représente l'hydrogène.

9. Composé de formule (II) suivant la revendication 7, dans lequel R³ représente le brome, R⁴ représente un groupe -C(O)R⁵ et R⁵ représente un groupe alkyle en C₁ à C₆.

10. Composé de formule (II) suivant la revendication 7, dans lequel R³ représente le brome, R⁴ représente un groupe -C (O) R⁵ et R⁵ représente un groupe -CH₃.

11. Procédé pour la préparation de composés de formule (II) 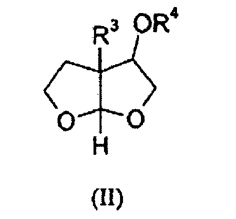 dans laquelle :
R³ représente un groupe halogéno ;
R⁴ représente l'hydrogène ou un groupe -C(O)R⁵ ;
R⁵ représente l'hydrogène, un groupe alkyle en C₁ à C₆, cycloalkyle en C₃ à C₈, aryle en C₆ à C₁₄ ou (aryle en C₆ à C₁₄) (alkyle en C₁ à C₆) ; et
de leurs diastéréoisomères, de leurs énantiomères, et de leurs mélanges, comprenant :
a) le traitement d'un composé de formule (XII) 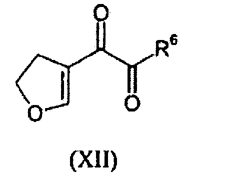 dans laquelle :
R⁶ représente un groupe halogéno, -OR⁷ ou -NR⁸R⁹ ;
R⁷ représente l'hydrogène, un groupe alkyle en C₁ à C₆, cycloalkyle en C₃ à C₈, aryle en C₆ à C₁₄ ou (aryle en C₆ à C₁₄) (alkyle en C₁ à C₆) ; et
R⁸ et R⁹ sont choisis indépendamment entre l'hydrogène, des groupes alkyle en C₁ à C₆, cycloalkyle en C₃ à C₈, aryle en C₆ à C₁₄ et (aryle en C₆ à C₁₄) (alkyle en C₁ à C₆) ;
avec un agent réducteur pour former un alcool de formule (III) 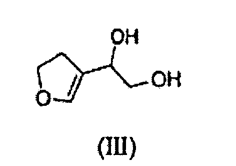
b) le traitement dudit alcool avec le brome, l'iode, le monochlorure d'iode, un N-fluoro-bis-sulfonamide ou un composé de formule (XIII) 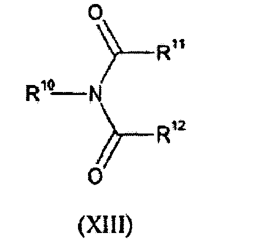 dans laquelle :
R¹⁰ représente le chlore, le brome ou l'iode ; et
R¹¹ et R¹² sont choisis indépendamment entre des groupes alkyle en C₁ à C₆, cycloalkyle en C₃ à C₈, aryle en C₆ à C₁₄ et (aryle en C₆ à C₁₄) (alkyle en C₁ à C₆), ou bien R¹¹ et R¹², conjointement avec les atomes auxquels ils sont fixés, forment un noyau penta- à octogonal ; pour former un composé de formule (II) dans lequel R³ représente un groupe halogéno et R⁴ représente l'hydrogène ; et
c) une résolution pour obtenir un composé de formule (II) dans lequel R³ représente un groupe halogéno, R⁴ représente l'hydrogène ou un groupe -C(O)R⁵, et R⁵ représente l'hydrogène, un groupe alkyle en C₁ à C₆, cycloalkyle en C₃ à C₈, aryle en C₆ à C₁₄ ou (aryle en C₆ à C₁₄) (alkyle en C₁ à C₆) .

12. Composé de formule (XIX)

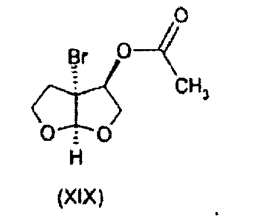

13. Procédé pour la préparation d'un composé de formule (XIX) 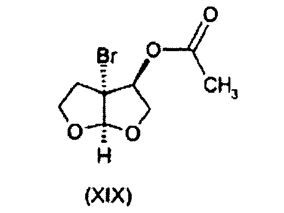 comprenant :
a) le traitement d'un composé de formule (XII) 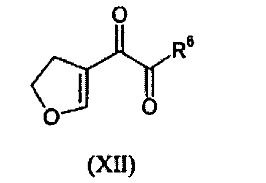 dans laquelle :
R⁶ représente un groupe halogéno, -OR⁷ ou -NR⁸R⁹ ;
R⁷ représente l'hydrogène, un groupe alkyle en C₁ à C₆, cycloalkyle en C₃ à C₈, aryle en C₆ à C₁₄ ou (aryle en C₆ à C₁₄) (alkyle en C₁ à C₆) ; et
R⁸ et R⁹ sont choisis indépendamment entre l'hydrogène, des groupes alkyle en C₁ à C₆, cycloalkyle en C₃ à C₈, aryle en C₆ à C₁₄ et (aryle en C₆ à C₁₄) (alkyle en C₁ à C₆) ;
avec un agent réducteur pour former un alcool de formule (III) 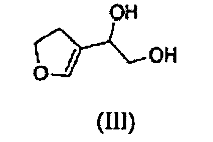
b) le traitement dudit alcool avec le brome, l'iode, le monochlorure d'iode, un N-fluoro-bis-sulfonamide ou un composé de formule (XIII) 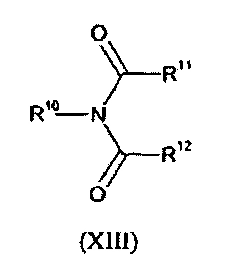 dans laquelle :
R¹⁰ représente le chlore, le brome ou l'iode ; et
_{R}¹¹ et _{R}¹² sont choisis indépendamment entre des groupes alkyle en C₁ à C₆, cycloalkyle en C₃ à C₈, aryle en C₆ à C₁₄ et (aryle en C₆ à C₁₄) (alkyle en C₁ à C₆), ou bien R¹¹ et R¹², conjointement avec les atomes auxquels ils sont fixés, forment un noyau penta- à octogonal ; et
c) facultativement une résolution pour obtenir un composé de formule (XIX) .

14. Composé de formule (XX)

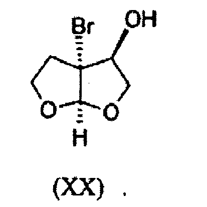

15. Procédé pour la préparation d'un composé de formule (XX) 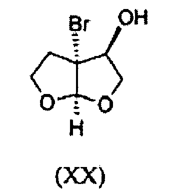 comprenant :
a) le traitement d'un composé de formule (XII) 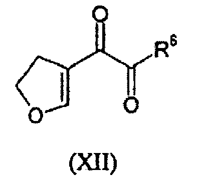 dans laquelle :
R⁶ représente un groupe halogéno, -OR⁷ ou -NR⁸R⁹;
R⁷ représente l'hydrogène, un groupe alkyle en C₁ à C₆, cycloalkyle en C₃ à C₈, aryle en C₆ à C₁₄ ou (aryle en C₆ à C₁₄) (alkyle en C₁ à C₆) ; et
R⁸ et R⁹ sont choisis indépendamment entre l'hydrogène, des groupes alkyle en C₁ à C₆, cycloalkyle en C₃ à C₈, aryle en C₆ à C₁₄ et (aryle en C₆ à C₁₄) (alkyle en C₁ à C₆) ;
avec un agent réducteur pour former un alcool de formule (III) 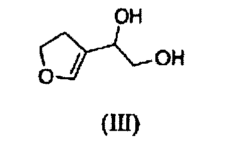
b) le traitement dudit alcool avec du N-bromosuccinimide pour former un composé de formule (XX) ; et
c) facultativement une résolution pour obtenir des diastéréoisomères des composés de formule (XX).

16. Composé de formule (III)

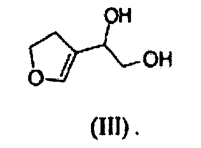

17. Procédé pour la préparation du composé (III) 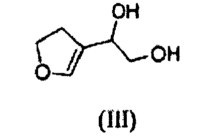 comprenant le traitement d'un composé de formule (XII) 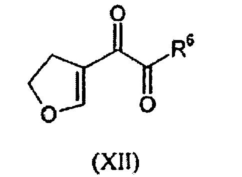 dans laquelle R⁶ représente un groupe halogéno, -OR⁷ ou -NR⁸R⁹ ; dans lequel R⁷ représente l'hydrogène, un groupe alkyle en C₁ à C₆, cycloalkyle en C₃ à C₈, aryle en C₆ à C₁₄ ou (aryle en C₆ à C₁₄) (alkyle en C₁ à C₆) ; et R⁸ et R⁹ sont choisis indépendamment entre l'hydrogène, des groupes alkyle en C₁ à C₆, cycloalkyle en C₃ à C₈, aryle en C₆ à C₁₄ et (aryle en C₆ à C₁₄) (alkyle en C₁ à C₆) ; avec un agent réducteur.

18. Procédé suivant la revendication 17, dans lequel l'agent réducteur est choisi dans le groupe consistant en hydrure de di-isobutylaluminium (DIBAL), borohydrure de sodium et hydrure de lithiumaluminium.

19. Procédé pour la préparation de composés de formule I, II, V, XIV, XIX et XX suivant l'une quelconque des revendications 1, 3, 4, 6, 11, 13 ou 15, dans lequel le premier agent réducteur est choisi dans le groupe consistant en hydrure de di-*iso*butylaluminium (DIBAL), borohydrure de sodium et hydrure de lithiumaluminium, R⁶ dans le composé de formule (XII) est un groupe -OR⁷ dans lequel R⁷ représente un groupe alkyle en C₁ à C₆, le composé de formule (XIII) étant le N-bromosuccinimide et le second agent réducteur étant le palladium sur du carbone en association avec de l'hydrogène.

20. Procédé suivant l'une quelconque des revendications 1, 2, 3 et 4, comprenant en outre l'étape de résolution pour obtenir des énantiomères uniques.
